(19) Europäisches Patentamt
European Patent Office
Office européen des brevets

(11) EP 3 138 918 A1

(12) **EUROPEAN PATENT APPLICATION**

(43) Date of publication:
**08.03.2017 Bulletin 2017/10**

(21) Application number: **15306362.3**

(22) Date of filing: **04.09.2015**

(51) Int Cl.:
***C12N 15/82*** *(2006.01)* ***C12N 15/52*** *(2006.01)*
***A01H 5/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **Genoplante-Valor**
**75015 Paris (FR)**

(72) Inventors:
- **HIREL, Bertrand**
  **78000 Versailles (FR)**
- **PEREZ, Pascual**
  **63450 Chanonat (FR)**

(74) Representative: **Gevers & Orès**
**41 avenue de Friedland**
**75008 Paris (FR)**

(54) **IMPROVEMENT OF PLANT YIELD**

(57) The invention relates to a method for improving the yield of a plant, comprising overexpressing in said plant a glutamine synthetase, said glutamine synthetase being both constitutively overexpressed in said plant and specifically overexpressed in the bundle sheath cells of said plant. The invention also relates to means for obtaining plants with improved yield.

EP 3 138 918 A1

## Description

The present invention relates to methods for controlling plant yield, in particular cereal crops, preferably maize crops, through the overexpression of glutamine synthetase (GS) activity.

The cereals including maize, wheat and rice account for 70% of worldwide food production. They require large quantities of nitrogenous fertilizers to attain maximal yields. In the past few years, there has been considerable interest in nitrogen use efficiency (NUE), which can be defined as the kernel yield per unit of nitrogen (N) in the soil and the N utilization efficiency (NutE), which is the yield per N taken up (Hirel and Lemaire, 2005a).

Using maize as a model crop, Hirel *et al.* (2005b) have investigated the changes in metabolite concentration and enzyme activities involved in N metabolism within a single leaf, at different stages of leaf growth and at different periods of plant development during the kernel-filling period. It was concluded that total N, chlorophyll, soluble protein content and GS activity are strongly interrelated and are indicators that mainly reflect the metabolic activity of individual leaves with regards to N assimilation and recycling, whatever the level of N fertilization.

Glutamine synthetase (GS; E.C.6.3.1.2) catalyzes the conversion of inorganic nitrogen (ammonium) into glutamine, according to the following reaction:

ATP + L-glutamate + $NH_3$ =>ADP + phosphate + L-glutamine.

All of the nitrogen in a plant, whether derived initially from nitrate, ammonium ions, N fixation, or generated by other reactions within the plant that release ammonium (decarboxylation of glycine during photorespiration, metabolism of N transport compounds and the action of phenylalanine ammonia lyase) is channelled through the reactions catalyzed by GS.

In maize, *Gln1-3* encodes the cytosolic GS isoenzyme GS1-3. The cDNA sequence of *Gln1-3* is available for instance in the GenBank database under the accession number X65928; the polypeptide sequence of GS1-3 is available for instance in the UniProtKB/Swiss-Prot database under the accession number P38561. *Gln1-4* encodes the cytosolic GS isoenzyme GS1-4. The cDNA sequence of *Gln1-4* is available for instance in the GenBank database under the accession number X65929; the polypeptide sequence of GS1-4 is available for instance in the UniProtKB/Swiss-Prot database under the accession number P38562. The amino acid sequences of GS1-3 and GS1-4 differ in that amino acid at position 41 is a serine in GS1-3 and a proline in GS1-4, and the amino acid at position 278 is an arginine in GS 1-3 and a lysine in GS 1-4.

*Gln1-3* gene plays a major part in the control of the number of kernels, while *Gln1-4* gene plays a major part in the control of the size of the kernels. *Gln1-3* and *Gln1-4* act specifically on grain production, without affecting vegetative biomass production (Martin *et al.,* 2006; International Application WO 2008/044150).

Overexpression of GS1-3 under the constitutive Cassava vein Mosaic Virus (CsVMV) promoter (Verdaguer *et al.,* 1996) in genetically transformed maize results in a significant increase in kernel yield, in particular in kernel number, under suboptimal nitrogen feeding conditions (Martin *et al.,* 2006; International Application WO 2008/044150). Expression of pCsVMV is constitutive and very high in mesophyll cells *in planta.*

He *et al.* (2014) have shown that overexpression of GS1-3 and GS1-4 under the constitutive *actin1* promoter in genetically transformed maize results in a significant increase in yield-related traits under sufficient nitrogen conditions.

It was proposed in International Application WO 2008/044150 to produce genetically transformed maize overexpressing GS1-3 under the control of the pCsVMV promoter in the mesophyll together with GS1-4 under the control of the maize rbcS promoter (Katayama *et al.,* 2000) in the bundle sheath cells in order to improve grain yield both in nitrogen-limiting and non-limiting conditions. However, International Application WO 2008/044150 does not describe the characterization of such a transgenic plant.

Despite the information available concerning the improvement of grain production in maize, as outlined above, there is still need for improving plant yield.

The inventors have shown in maize that the overexpression of the same glutamine synthetase of maize downstream two different promoters - a constitutive promoter and a bundle sheath cell specific promoter - leads to yield improvement. More specifically, the sequence used by the inventors is SEQ ID NO: 1 (referred to as ZmGS1-b). ZmGS1-b differs from ZmGS1-3 because of three amino acids. ZmGlnl-b cDNA of SEQ ID NO: 5 encodes the ZmGS1-b enzyme.

Accordingly, the present invention provides a method for improving the yield of a plant, wherein said method comprises overexpressing in said plant a glutamine synthetase having at least 90% identity, or by order of increasing preference at least 91%, 92%, 93%, 94%, 95%, 96%, 97%, 98%, 99% or 100% identity, with the polypeptide of SEQ ID NO: 1 (ZmGS1-b) and said glutamine synthetase being both constitutively overexpressed in said plant and specifically overexpressed in the bundle sheath cells of said plant, and provided that said glutamine synthetase constitutively overexpressed and said glutamine synthetase specifically overexpressed have the same amino acid sequence.

Unless otherwise specified, the percentage of identity between two sequences which are mentioned herein are calculated from an alignment of the two sequences over their whole length. One can use the BLAST program (Tatusova and Madden TL, 1999) with the default parameters (open gap penalty = 2; extension gap penalty = 5; matrix = BLOSUM 62).

The glutamine synthetase as defined above encompasses recombinant, synthetic or chimeric enzymes.

The glutamine synthetase as defined above can be from a plant of the same species as said plant in which the glutamine synthetase is overexpressed.

Advantageously, said glutamine synthetase is an autologous glutamine synthetase of said plant.

In a preferred embodiment, the glutamine synthetase is from a plant selected from the group consisting of: *Zea mays, Setaria italica, Saccharum officinarum, Oryza sativa, Oryza brachyantha, Oryza glaberrima, Brachypodium distachyon, Hordeum vulgare, Triticum aestivum, Secale cereale, Secale cereale x Triticum turgidum, Lolium perenne* and *Aegilops tauschii,* preferably *Zea mays.*

Advantageously the glutamine synthetase is an orthologous of ZmGS1-b (SEQ ID NO: 1) selected from the group consisting of SEQ ID NO: 9 to SEQ ID NO: 26, preferably SEQ ID NO: 9 to SEQ ID NO: 14 (glutamine synthetases from *Zea mays*).

As used herein the term "yield" refers to the amount of product harvested from a given acreage (e.g., weight of seeds per unit area). It is often expressed in metric quintals (1 q = 100 kg) per hectare in the case of cereals. By way of example, in maize, kernel number, kernel size, kernel weight, ear length, ear diameter and ear weight are maize yield-related traits.

According to the method of the present invention the yields (e.g., grain yields when the plant is maize) are improved in said plant, both in N-limiting and non-limiting conditions.

In a preferred embodiment, the constitutively overexpressed glutamine synthetase is constitutively overexpressed in the mesophyll of said plant.

Bundle sheath cells are parenchymatous cells which surround the vascular bundle in plants. They are particularly well identified in C4 plants because of their significant role in the C4 pathway but they were also identified in C3 plants (Leegood *et al.,* 2008). Typically, enzymes associated with sulfur assimilation are most abundant in bundle sheath cells. In addition, the p-subunit of glycine decarboxylase (GDC) is also expressed in bundle sheath cells. See for review: Berry *et al.,* 2008; John *et al.,* 2014.

In a preferred embodiment, the method according to the present invention applies to C4 plants.

C4 plants are well known in the art. In C4 plants, the initial fixation of atmospheric carbon dioxide occurs on phosphoenol pyruvate (a C3 compound) and is catalysed by the enzyme phosphoenolpyruvate carboxylase (PEPC). This reaction results in the formation of oxaloacetic acid (a C4 acid), hence the name C4 plants (see for review Berry *et al.,* 2008).

Preferred C4 plants include maize (*Zea mays*), sorghum, sugar cane, millet and miscanthus, more preferably maize, sorghum, sugar cane, most preferably maize.

The method according to the present invention can also apply to C3 plants, such as wheat (*Triticum aestivum, Triticum durum*), rice and barley.

The term "overexpressing" a glutamine synthetase in a plant, herein refers to artificially increasing the quantity of said active GS enzyme produced in said plant compared to a reference plant.

A preferred method for overexpressing a glutamine synthetase comprises introducing into the genome of said plant a DNA construct comprising a nucleotide sequence encoding said glutamine synthetase, placed under the control of appropriate promoters.

The instant invention also provides means for carrying out said overexpression.

This includes in particular recombinant DNA constructs for expressing a glutamine synthetase as defined above in a host-cell, or a host organism, in particular a plant cell or a plant. These DNA constructs can be obtained and introduced in said host cell or organism by the well-known techniques of recombinant DNA and genetic engineering.

Recombinant DNA constructs of the invention include in particular an isolated polynucleotide comprising a recombinant expression cassette comprising a polynucleotide encoding a glutamine synthetase under the control of an heterologous constitutive promoter functional in a plant cell as defined above and a recombinant expression cassette comprising a polynucleotide encoding said glutamine synthetase under the control of a bundle sheath cell specific promoter, provided that both glutamine synthetase enzymes have the same amino acid sequence.

The heterologous promoter of the invention is a promoter functional in a plant cell, *i.e.,* capable of directing transcription of a polynucleotide encoding a glutamine synthetase, as defined above, in a plant cell.

A large choice of promoters suitable for expression of genes in plants, and in particular in maize, is available in the art. They can be obtained for instance from plants, plant viruses, or bacteria such as *Agrobacterium.*

Constitutive promoters are promoters which are active in most tissues and cells, in particular in mesophyll cells, and under most environmental conditions. Non-limitative examples of constitutive promoters that are commonly used are the cauliflower mosaic virus (CaMV) 35S promoter, the 19S promoter (Kay *et al.,* 1987), the Cassava vein Mosaic Virus (CsVMV) promoter (Verdaguer *et al.,* 1996), the rice actin promoter followed by the rice actin intron (RAP-RAI; contained in the plasmid pAct1-F4) (McElroy *et al.,* 1991), the pCRV promoter (Depigny-This *et al.,* 1992), the ubiquitin 1 promoter of maize (Christensen *et al.,* 1996) and the regulatory sequences of the T-DNA of *Agrobacterium tumefaciens,* including the mannopine synthase promoter, the nopaline synthase (Nos) promoter and the octopine synthase promoter.

Cell specific promoters are active only or mainly in certain cell types. Non-limitative examples of bundle sheath cell specific promoters that can be used in the present invention include for instance the Rubisco small subunit (rbcS) promoter (Katayama *et al.,* 2000; SEQ ID NO: 8) or the glycine decarboxylase (GDC) p-subunit promoter (Engelmann *et al.,* 2008; SEQ ID NO: 27).

According to a preferred embodiment, the constitutive promoter is the Cassava vein Mosaic Virus (CsVMV) promoter (e.g., SEQ ID NO: 3) and the bundle sheath cell specific promoter is the Rubisco small subunit (rbcS) promoter (e.g., SEQ ID NO: 8).

The expression cassettes generally also include a transcriptional terminator, such as the 35S transcriptional terminator or Nos terminator (Depicker *et al.,* 1982). They may also include other regulatory sequences, such as transcription enhancer sequences.

By way of example a recombinant DNA construct according to the present invention comprises the ZmGlnl-b cDNA flanked by the Cassava vein mosaic virus promoter (pCsVMV) linked to an actin intron, the ZmGln1-b terminator and the 3'Nos terminator, and another copy of the ZmGln1-b cDNA flanked by the rbcS promoter, the terminator ZmGlnl-b and the 3'Nos terminator. This DNA construct is described in SEQ ID NO: 2.

Recombinant DNA constructs of the invention also include recombinant vectors containing a recombinant expression cassette comprising a polynucleotide encoding a glutamine synthetase under the control of an heterologous constitutive promoter functional in a plant cell (*e.g.* the CsVMV promoter) as defined above and a polynucleotide encoding said glutamine synthetase under the control of a bundle sheath cell specific promoter (e.g., the rbcS promoter), as defined above.

In a particular embodiment, the heterologous constitutive promoter functional in a plant cell is pCsVMV of SEQ ID NO: 3 and the bundle sheath cell specific promoter is proZmRbcS of SEQ ID NO: 8.

Recombinant vectors of the invention may also include other sequences of interest, such as, for instance, one or more marker genes, which allow for selection of transformed hosts.

Advantageously, the selectable marker gene is comprised between two Ds elements (*i.e.,* transposons) in order for its removal at a later stage by interacting with the Ac transposase. This elimination system is known from one skilled in the art. By way of example, it has been described in Goldsbrough *et al.,* 1993.

The selection of suitable vectors and the methods for inserting DNA constructs therein are well known to persons of ordinary skill in the art. The choice of the vector depends on the intended host and on the intended method of transformation of said host. A variety of techniques for genetic transformation of plant cells or plants are available in the art for many plant species. By way of non-limitative examples, one can mention virus-mediated transformation, transformation by microinjection, by electroporation, microprojectile-mediated transformation, *Agrobacterium* mediated transformation (Ishida *et al.,* 1996), and the like.

The invention also comprises host cells containing a recombinant DNA construct of the invention. These host cells can be prokaryotic cells or eukaryotic cells, in particular plant cells, and preferably maize cells.

The invention also provides a method for producing a transgenic plant, in particular a maize plant, having an improved yield. Said method comprises transforming a plant cell *(e.g.,* a maize cell) by a DNA construct of the invention and regenerating from said plant cell *(e.g.,* maize cell) a transgenic plant *(e.g.,* maize plant) overexpressing a glutamine synthetase under the control of a heterologous constitutive promoter functional in a plant cell as defined above and said glutamine synthetase under the control of a bundle sheath cell specific promoter, as defined above.

According to a preferred embodiment or the method of the invention, it comprises transforming a plant cell, in particular a maize cell, by a recombinant vector of the invention comprising a polynucleotide encoding a glutamine synthetase under the control of an heterologous constitutive promoter functional in a plant cell as defined above, and by a recombinant vector of the invention comprising a polynucleotide encoding said glutamine synthetase under the control of a bundle sheath cell specific promoter, as defined above, and regenerating from said plant cell *(e.g.,* maize cell) a transgenic plant *(e.g.,* maize plant) overexpressing both glutamine synthetase enzymes.

According to another preferred embodiment or the method of the invention, it comprises transforming a plant cell, in particular a maize cell, by a recombinant vector of the invention comprising a polynucleotide encoding a glutamine synthetase under the control of an heterologous constitutive promoter functional in a plant cell as defined above and a polynucleotide encoding said glutamine synthetase under the control of a bundle sheath cell specific promoter, as defined above, and regenerating from said plant cell *(e.g.,* maize cell) a transgenic plant (e.g., maize plant) overexpressing both glutamine synthetase enzymes.

The invention also comprises plants genetically transformed by a recombinant DNA construct of the invention, and overexpressing a glutamine synthetase under the control of a heterologous constitutive promoter functional in a plant cell as defined above and said glutamine synthetase under the control of a bundle sheath cell specific promoter, as defined above. Preferably, said plants are transgenic maize plants, obtainable by a method of the invention, overexpressing a glutamine synthetase under the control of a heterologous constitutive promoter functional in a plant cell as defined above and said glutamine synthetase under the control of a bundle sheath cell specific promoter, as defined above. In said transgenic plants a DNA construct of the invention is comprised in a transgene integrated (*i.e.,* stably integrated) in the plant genome, so that it is passed onto successive plant generations. Thus the transgenic plants of the invention include not only the plants resulting from the initial transgenesis, but also their descendants, as far as they contain a recombinant DNA construct of the invention. The overexpression of a glutamine synthetase under the control of a heterologous constitutive promoter functional in a plant cell as defined above and said glutamine synthetase under the control of a bundle sheath cell specific promoter, as defined above in said plants provides them with an improved yield, when compared with a plant devoid of said transgene(s).

Accordingly, the invention provides a transgenic plant or an isolated organ or tissue thereof comprising, stably integrated in its genome, a recombinant expression cassette comprising a polynucleotide encoding a glutamine synthetase under the control of a heterologous constitutive promoter functional in a plant cell as defined above and a polynucleotide encoding said glutamine synthetase under the control of a bundle sheath cell specific promoter, as defined above.

The invention also encompasses isolated organs or tissues of said transgenic plants (such as seeds, leafs, flowers, roots, stems, ears) containing a recombinant expression cassette of the invention.

Foregoing and other objects and advantages of the invention will become more apparent from the following detailed description and accompanying drawings. It is to be understood however that this foregoing detailed description is exemplary only and is not restrictive of the invention.

**Figure 1:** Schematic representation of the T-DNA fragment introduced into maize plants. RB and LB represent the right and left borders of the T-DNA. Ds (3'Ac) and Ds (5'Ac) represent the Ds transposable elements used to further remove the selection marker conferring resistance to kanamycin. NptII is the neomycin phosphotransferase gene with the Actin promoter and the Actin intron and nopaline synthetase terminator (3'Nos) conferring kanamycin resistance. An Actin intron was placed between the maize ZmGln1-b cDNA and the CsVMV promoter.

**Figure 2:** Western-blot **(A)** using tobacco antibodies recognizing GS1 and GS2, or enzymatic activity **(B)** analyses on leaf or root samples. The upper band seen on the western-blot corresponds to the plastidic GS (GS2) subunit, and the lower band corresponds to the cytosolic GS (GS1) subunit. **(A)** Western-blot analysis of GS1-b transformed maize lines (LXX) or from corresponding hybrid lines (HYY), leaf samples. **(B)** Enzymatic assays with samples of GS1-b transformed maize lines. A188: untransformed control line HA188: corresponding hybrid from untransformed A188 line.

**Figure 3:** Yield data and grain humidity of GS1-b over-expressing lines. The yield data is expressed as percentage of the control lines and has been normalized to grain moisture at 15%. The larger symbols correspond to the average value obtained with all transformed lines (large triangle) or with the control lines (large square). For each data point calculated, the corresponding p-value is also indicated by a cross. The p-value scale is on the left side of the figure.

**Figure 4:** Kernel number per ear and yield data of GS1-b over-expressing lines. The kernel number per ear is expressed as percentage of the control lines. The yield data, normalized to grain moisture at 15%, is expressed as quintal per hectare.

## EXAMPLE: OVEREXPRESSION OF GS1-b (SEQ ID NO: 1) IN LEAF MESOPHYLL AND BUNDLE SHEATH CELLS IN MAIZE

The strategy, described below, is to transform maize with a T-DNA expressing a copy of the Gln1-b cDNA under control of the pCsVMV promoter, together with another copy of the Gln1-b cDNA under control of the maize bundle sheath cell specific promoter of the Rubisco (rbcS) gene (Katayama *et al.,* 2000), in order to obtain transgenic maize plants expressing GS1-b both in the mesophyll and in the bundle sheath cells.

### Plant transformation, regeneration and characterization

Maize transformation of the inbred line A188 with *Agrobacterium tumefaciens* strain LBA4404 harbouring a super-binary plasmid was performed essentially as described by Ishida *et al.,* 1996. In particular, the composition of all media cited hereafter is detailed in this reference. The protocol was slightly modified concerning the selective marker, which was the NPTII gene instead of the bar gene.

### Super-binary plasmid pRec 1459

The super-binary plasmid used for transformation was the result of a recombination between plasmid pBIOS 1459 and the plasmid pSB1 (harbouring the virB and virG genes isolated from the super-virulent strain A281) within the *Agrobacterium* strain LBA4404 (pSB1) (Komari *et al.,* 1996) forming the plasmid pRec 1459. pBIOS 1459 is a derivative of pSB11 (Komari *et al.,* 1996) harbouring between the T-DNA borders, a neomycin resistance cassette (NPTII gene) (Bevan *et al.,* 1992; Berg and Berg, 1983) flanked by an actin promoter (McElroy *et al.,* 1990) and 3'Nos terminator (Depicker *et al.,* 1982;), and the ZmGlnl-b cDNA (Sakakibara *et al.,* 1992, SEQ ID NO: 5) flanked by the Cassava vein mosaic virus promoter (pCsVMV) (Verdaguer *et al.,* 1996; SEQ ID NO: 3) linked to an actin intron (McElroy *et al.,* 1990; SEQ ID NO: 4), the ZmGlnl-b terminator (SEQ ID NO: 6) and 3'Nos terminator (SEQ ID NO: 7), and another copy of the ZmGln1-b cDNA (SEQ ID NO: 5) flanked by the rbcS promoter (SEQ ID NO: 8), the ZmGlnl-b terminator (SEQ ID NO: 6) and 3'Nos terminator (SEQ ID NO: 7). The resulting nucleic sequence proCsVMV-OsActin_intron- ZmGlnl-b -terminator ZmGln1-b -terminator NOS-proZmRbcS- ZmGlnl-b -terminator ZmGln1-b -terminator NOS is referred to as SEQ ID NO: 2. The resulting agrobacterial strain used for transformation was LB4404 (pRec 1459). pRec 1459 is schematized in Figure 1.

## Transgenic plants

Plant transformation was conducted using immature maize embryos isolated at 10 days after pollination. Immature embryos were incubated for 5 min with *A. tumefaciens* and cultured for 3 days on a LSAs medium without antibiotic selection in the dark at 25°C. Upon transfer to the LSD5 medium, *A. tumefaciens* was counter-selected by the presence of 250 mg L-1 cefotaxime, and the transformed *calli* were selected by the presence of 50 mg mL-1 kanamycin. After 2 weeks of culture, developing *calli* were transferred to LSD10 medium containing 50 mg mL-1 kanamycin and grown for 3 weeks. Type I *calli* were excised and cultured for another 3 weeks on kanamycin. For regeneration, well-developed type I *calli* were cultured on LSZ medium at 22°C under continuous selective pressure and on kanamycin. After 2 weeks, *calli* bearing shoots were transferred to RMG2 medium and cultured another 2 weeks to allow the development of roots before the transfer of the plantlets to soil and gradual acclimatization to ambient humidity. Plants were then cultivated in a glasshouse (18°C-24°C) and selfed or pollinated with line A188 to produce seeds.

A number of transgenic lines were selected and tested by Quantitative PCR for the number of inserted T-DNA copies and by conventional PCR for the integrity of the inserted T-DNA. Only plants with up to 2 full length T-DNA copies were kept for further analysis.

## Biochemical characterization of transgenic plants over-expressing GS1-b

The presence of the introduced GS1-b protein was determined by western-blot (Figure 2A) using tobacco antibodies recognizing GS1 and GS2, or enzymatic activity (Figure 2B) analyses on leaf or root samples of greenhouse grown plants.

It can be seen that leaves of GS 1-b transformed lines contain a higher level of GS1 proteins, as determined by western-blot analysis, and that GS1 enzymatic activity levels are higher (more than a 2 fold increase) than those of control lines. The same results hold true for the derived hybrids.

## Field trials

Hybrids with a tester line were obtained from T3 plants issued from the selected GS1-b over-expressing transgenic maize lines.

The transformant (TO) plants were first crossed with the A188 line thereby producing T1 plants. T1 plants were then self-pollinated twice, producing T3 plants which are homozygous lines containing the transgene. These T3 plants were then crossed with the tester line thereby leading to a hybrid. This hybrid is at a T4 level with regards to the transformation step and is hemizygous for the transgene. These hybrid plants are used in field experiments.

Control hybrids are obtained as follows:

Control Equiv corresponds to a cross between A188 line (the line used for transformation) and the tester line.

Control HNS corresponds to a cross between a null segregant (isolated after the second self-pollination of the T1 plants) and the tester line. Said null segregant is a homozygous line which does not bear the transgene. Although the null segregant theoretically presents the same genome as A188, it has undergone *in vitro* culture (via the steps of callus differentiation and regeneration) and may thus present mutations (either genetic or epigenetic) with regards to an A188 line that has not undergone *in vitro* culture.

Yield was calculated as follows:

During harvest, grain weight and grain moisture are measured using an on-board equipment on the combine harvester. Grain weight is then normalized to moisture at 15 %, using the following formula:

Normalized grain weight = measured grain weight x (100 - measured moisture (as a percentage)) / 85 (which is 100 - normalized moisture at 15 %).

As an example, if the measured grain moisture is 25 %, the normalized grain weight will be: normalized grain weight = measured grain weight x 75 / 85.

Yield is then expressed in a conventional unit (such as quintal per hectare).

Experimental design:

The experimental block comprised 5 replicates. Each replicate comprised about 58.5 plants per plot at a density of 69600 plants/ha.

Two controls were used in this experiment as described above (a null segregant (HNS) and a control equivalent (A188 crossed with the tester line).

Grain moisture, thousand kernel weight yield and ear kernel number data are represented in the Table 1 below.

Table 1: Grain moisture, TKW yield and ear kernel number measured for different transformation events with the ZmGln1-b cDNA. Data is given as *per se* or as a percentage compared to the control sample.

| Sample | Grain moisture (%) | Grain moisture as % of control average | P-value compared to the control average | Thousand Kernel Weight (TKW) (g) | TKW as % of control average | P-value compared to the control average | Yield (Qx/Ha) | Yield as % of control average | P-value compared to the control average | Number of Kernels per ear | Kernel number per ear as % of control average | P-value compared to the control average |
|---|---|---|---|---|---|---|---|---|---|---|---|---|
| Control Equiv | 19,3 | | | 267,8 | | | 64,8 | | | 480 | | |
| T01617_027 | 19,1 | 99 | 0,7366 | 284,4 | 103 | 0,401 | 71,9 | 111 | 0,012 | 519 | 106 | 0,0679 |
| T01617_022 | 19,3 | 100 | 0,8984 | 267,6 | 97 | 0,313 | 70,2 | 108 | 0,055 | 500 | 102 | 0,4971 |
| T01617_032 | 18,8 | 97 | 0,2621 | 272,0 | 98 | 0,598 | 69,8 | 108 | 0,075 | 544 | 111 | 0,0008 |
| T01617_020 | 18,8 | 97 | 0,2431 | 279,0 | 101 | 0,806 | 69,7 | 108 | 0,079 | 507 | 104 | 0,2527 |
| T01617_023 | 18,8 | 97 | 0,2621 | 282,8 | 102 | 0,507 | 68,4 | 106 | 0,194 | 525 | 108 | 0,0294 |
| T01617_014 | 18,6 | 96 | 0,1044 | 283,4 | 102 | 0,465 | 67,5 | 104 | 0,338 | 495 | 101 | 0,7142 |
| T01617_017 | 18,9 | 98 | 0,3727 | 284,2 | 103 | 0,413 | 66,7 | 103 | 0,495 | 501 | 103 | 0,4316 |
| T01617_031 | 19,0 | 99 | 0,5700 | 270,6 | 98 | 0,496 | 66,3 | 102 | 0,596 | 487 | 100 | 0,9554 |
| T01617_018 | 19,1 | 99 | 0,7366 | 274,8 | 99 | 0,827 | 66,2 | 102 | 0,607 | 504 | 103 | 0,3380 |
| T01617_012 | 19,3 | 100 | 0,8984 | 279,6 | 101 | 0,755 | 65,0 | 100 | 0,935 | 501 | 103 | 0,4328 |
| Control HNS | 19,3 | | | 285,8 | | | 64,8 | | | 497 | | |
| Control average | 19,3 | 100 | | 276,8 | | | 64,8 | 100 | | 488 | 100 | |
| T01617 average | 19,0 | 98 | | 277,8 | | | 68,2 | 105 | 0,041 | 508 | 104 | 0,0417 |

Figure 3 represents the yield expressed as percentage of the control samples and in function of grain moisture.

Improved yield (between 5 and 10% improvement compared to the controls) was observed for the hybrid plants of most GS1-b over-expressing lines. Grains from GS1-b over-expressor plants were also less humid than grains from control plants (in average 19% vs 19.3%). The transgenic lines can be grouped by those leading to a yield increase (large circle) and those given similar values to the control samples (small circle).

Figure 4 represents the ear kernel number expressed as percentage of the control samples and in function of yield.

Improved yield may thus be explained by the increased number of kernels per ear of the transgenic plants (see Figure 4), since a correlation between yield and ear kernel number can be seen.

## REFERENCES

Berg and Berg, Biotechnol., 1: 417-435, 1983.

Berry et al., Kranz Anatomy and the C4 Pathway, In: Encyclopedia of Life Sciences. John Wiley & Sons, Ltd: Chichester, 2008.

Bevan et al., Biotechnol., 24: 367-70, 1992.

Christensen et al., Transgenic. Res., 5: 213, 1996.

Depicker et al., J. Mol. Appl. Genet., 1: 561-73, 1982.

Depigny-This et al., Plant Molecular Biology, 20: 467-479, 1992.

Engelmann et al., Plant Physiol, 246: 1773-1785, 2008.

Goldsbrough et al., Nature Biotechnology, 11: 1286-1292, 1993.

He et al., Maydica electronic publication, 59: 250-256, 2014.

Hirel and Lemaire, A. Basra, and S. Goyal, eds. (Haworth's Food Product Press. Binghamton, New-York), 15: 213-257, 2005a.

Hirel et al., Physiol. Plant, 124: 178-188, 2005b.

Ishida et al., Nat. Biotechnol., 14: 745-750, 1996.

John et al., Plant Physiol, 165: 62-75, 2014

Katayama et al., Plant Mol. Biol., 44: 99-106, 2000.

Kay et al., Science, 236: 1299-1302, 1987.

Komari et al., Plant J., 10: 165-74, 1996.

Leegood et al., Journal of Experimental Botany, 59 : 1663-1673, 2008.

Martin et al., Plant Cell, 18: 3252-74, 2006.

McElroy et al., Plant Cell, 2: 163, 1990.

McElroy et al., Mol. Gen. Genet., 231: 150-160, 1991.

Sakakibara et al., Plant Cell Physiol., 33: 1193-1198, 1992.

Tatusova and Madden,. FEMS Microbiol. Lett., 174: 247-250, 1999.

Verdaguer et al., Plant Mol. Biol., 6: 1129-39, 1996.

## Claims

1. A method for improving the yield of a plant, wherein said method comprises overexpressing in said plant a glutamine synthetase having at least 90% identity with the polypeptide of SEQ ID NO: 1 and, said glutamine synthetase being both constitutively overexpressed in said plant and specifically overexpressed in the bundle sheath cells of said plant, and provided that said glutamine synthetase constitutively overexpressed and said glutamine synthetase specifically overexpressed have the same amino acid sequence.

2. The method according to claim 1, wherein said glutamine synthetase is from a plant selected from the group consisting of *Zea mays, Setaria italica, Saccharum officinarum, Oryza sativa, Oryza brachyantha, Oryza glaberrima, Brachypodium distachyon, Hordeum vulgare, Triticum aestivum, Secale cereale, Secale cereale x Triticum turgidum, Lolium perenne* and *Aegilops tauschii,* preferably *Zea mays.*

3. The method according to claim 1 or claim 2, wherein said glutamine synthetase is selected from the group consisting of SEQ ID NO: 1 and SEQ ID NO: 9 to SEQ ID NO: 26, preferably SEQ ID NO: 1 and SEQ ID NO: 9 to SEQ ID NO: 14.

4. The method according to any one of claims 1 to 3, wherein said plant is a C4 plant preferably selected from the group consisting of maize, sorghum, sugar cane, millet and miscanthus and most preferably maize.

5. The method according to claim 1, wherein said plant is maize and said glutamine synthetase has the amino acid sequence SEQ ID NO: 1 (ZmGS1-b).

6. An isolated polynucleotide comprising a recombinant expression cassette comprising a polynucleotide encoding a glutamine synthetase as defined in any one of claims 1 to 5 under the control of an heterologous constitutive promoter functional in a plant cell and a recombinant expression cassette comprising a polynucleotide encoding a glutamine synthetase as defined in any one of claims 1 to 5 under the control of a bundle sheath cell specific promoter, provided that both glutamine synthetase enzymes have the same amino acid sequence.

**7.** The isolated polynucleotide comprising a recombinant expression cassette according to claim 6 wherein the heterologous constitutive promoter functional in a plant cell is SEQ ID NO: 3 (pCsVMV) and the bundle sheath cell specific promoter is SEQ ID NO: 8 (proZmRbcS).

**8.** A recombinant vector comprising a polynucleotide of claim 6 or claim 7.

**9.** A host cell comprising:

- a polynucleotide of claim 6 or claim 7,
- a recombinant expression cassette comprising a polynucleotide encoding a glutamine synthetase as defined in any one of claims 1 to 5 under the control of an heterologous constitutive promoter functional in a plant cell and a recombinant expression cassette comprising a polynucleotide encoding a glutamine synthetase as defined in any one of claims 1 to 5 under the control of a bundle sheath cell specific promoter, provided that both glutamine synthetase enzymes have the same amino acid sequence, or
- a recombinant vector of claim 8.

**10.** The host cell according to claim 9 which is a plant cell, preferably a C4 plant cell.

**11.** The host cell according to claim 10 which is a maize cell.

**12.** A method for producing a transgenic plant having an improved seed yield, wherein said method comprises:

- providing a plant cell of claim 10 or 11;
- regenerating from said plant cell a transgenic plant overexpressing a glutamine synthetase as defined in any one of claims 1 to 5.

**13.** The method according to claim 12, wherein the transgenic plant is a C4 plant, preferably a maize plant.

**14.** A transgenic plant, or an isolated organ or tissue thereof comprising, stably integrated in its genome, a recombinant expression cassette comprising a polynucleotide encoding a glutamine synthetase as defined in any one of claims 1 to 5 under the control of an heterologous constitutive promoter functional in a plant cell and a recombinant expression cassette comprising a polynucleotide encoding a glutamine synthetase as defined in any one of claims 1 to 5 under the control of a bundle sheath cell specific promoter, provided that both glutamine synthetase enzymes have the same amino acid sequence.

**15.** The transgenic plant according to claim 14, wherein said plant is a C4 plant, preferably a transgenic maize plant.

**16.** Seed or kernel containing a recombinant expression cassette comprising a polynucleotide encoding a glutamine synthetase as defined in any one of claims 1 to 5 under the control of an heterologous constitutive promoter functional in a plant cell and a recombinant expression cassette comprising a polynucleotide encoding a glutamine synthetase as defined in any one of claims 1 to 5 under the control of a bundle sheath cell specific promoter, provided that both glutamine synthetase enzymes have the same amino acid sequence, obtained from a transgenic plant of claim 14 or claim 15.

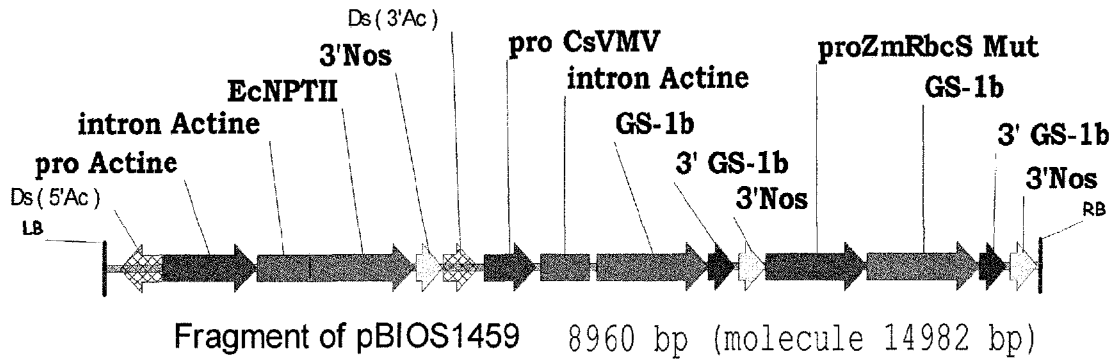


**Figure 1**

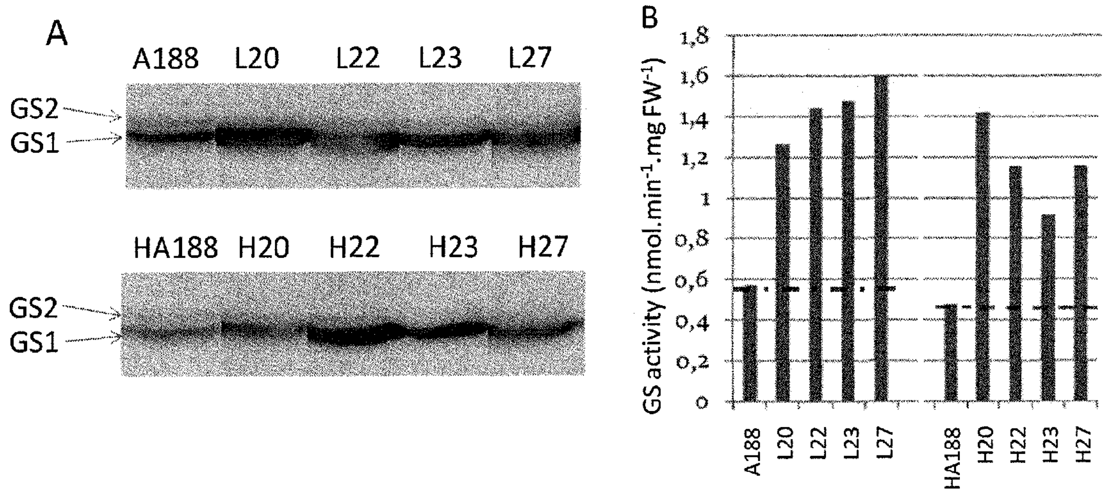


**Figure 2**

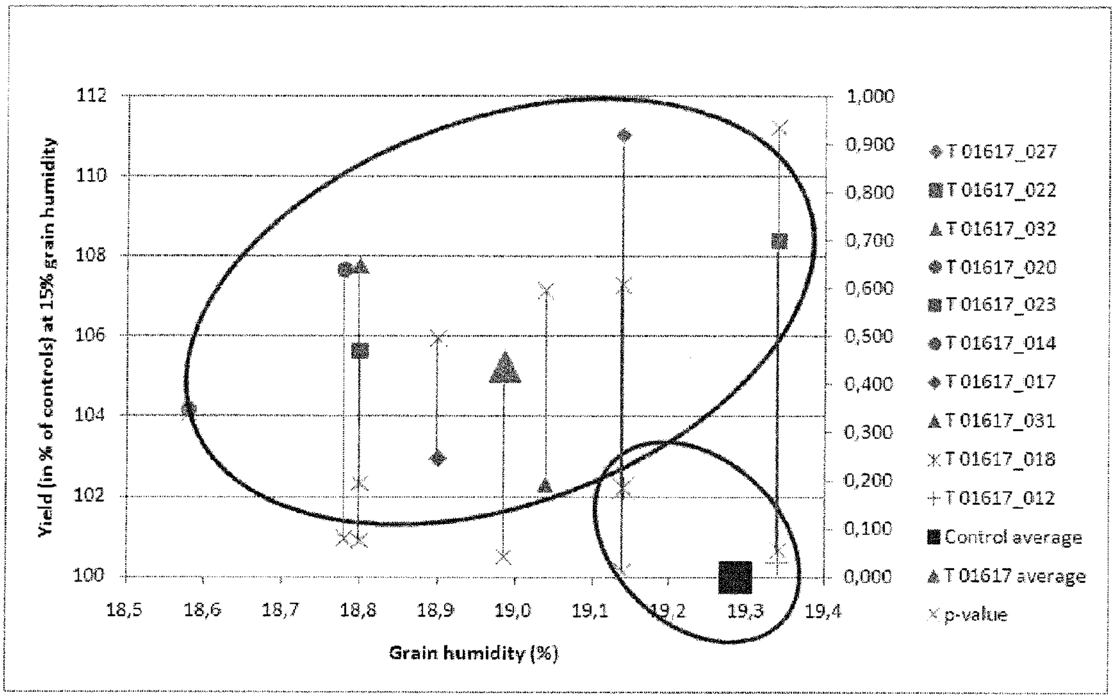


Figure 3

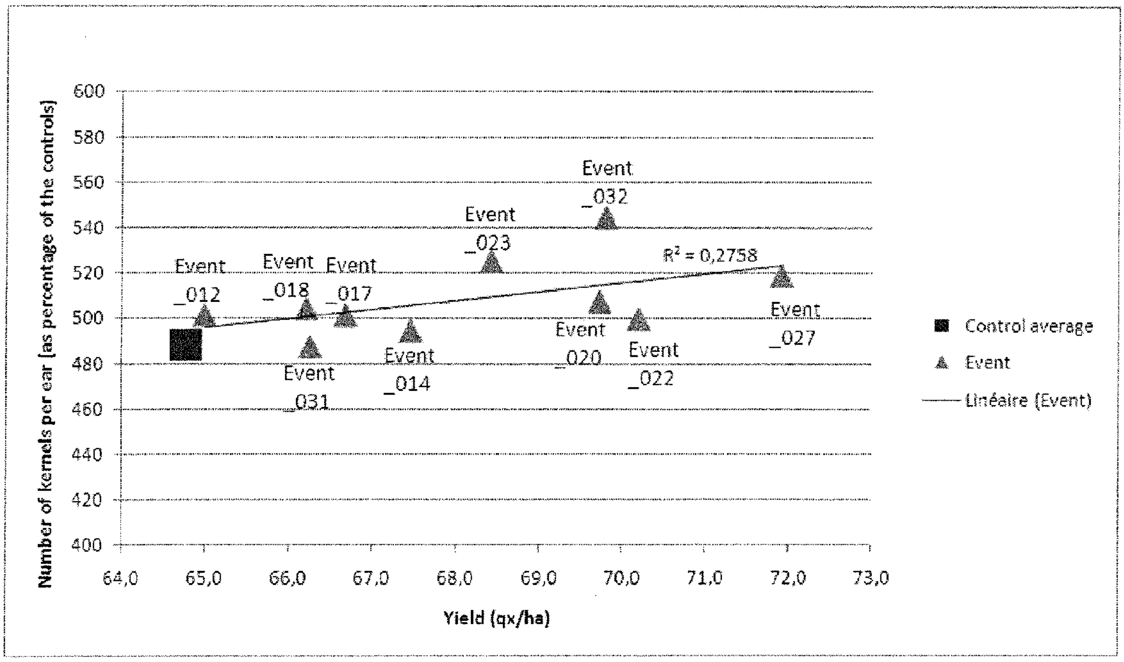


Figure 4

Europäisches Patentamt
European Patent Office
Office européen des brevets

# EUROPEAN SEARCH REPORT

Application Number
EP 15 30 6362

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2008/044150 A2 (GENOPLANTE VALOR [FR]; HIREL BERTRAND [FR]; PEREZ PASCUAL [FR]) 17 April 2008 (2008-04-17) * page 5, line 22 - line 27; example 6 * | 1-16 | INV. C12N15/82 C12N15/52 A01H5/00 |
| A | WO 2011/088065 A1 (MONSANTO TECHNOLOGY LLC [US]; ALLEN EDWARDS [US]; ANIL VEENA S [IN]; B) 21 July 2011 (2011-07-21) * claims 1-15; sequence 58 * | 1-16 | |
| A | DATABASE UniProt [Online] 23 September 2008 (2008-09-23), "RecName: Full=Glutamine synthetase {ECO:0000256¦RuleBase:RU004356}; EC=6.3.1.2 {ECO:0000256¦RuleBase:RU004356};", XP002754005, retrieved from EBI accession no. UNIPROT:B4G1P1 Database accession no. B4G1P1 * sequence * | 1-16 | |

TECHNICAL FIELDS SEARCHED (IPC)

C12N
A01H

The present search report has been drawn up for all claims

1

| Place of search | Date of completion of the search | Examiner |
|---|---|---|
| The Hague | 8 February 2016 | Maddox, Andrew |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 30 6362

This annex lists the patent family members relating to the patent documents cited in the above-mentioned European search report. The members are as contained in the European Patent Office EDP file on
The European Patent Office is in no way liable for these particulars which are merely given for the purpose of information.

08-02-2016

| Patent document cited in search report | Publication date | Patent family member(s) | Publication date |
|---|---|---|---|
| WO 2008044150 A2 | 17-04-2008 | AU 2007306040 A1 | 17-04-2008 |
| | | CA 2665739 A1 | 17-04-2008 |
| | | CN 101553570 A | 07-10-2009 |
| | | EP 1911847 A1 | 16-04-2008 |
| | | EP 2078088 A2 | 15-07-2009 |
| | | EP 2636745 A1 | 11-09-2013 |
| | | US 2010107280 A1 | 29-04-2010 |
| | | US 2013185829 A1 | 18-07-2013 |
| | | WO 2008044150 A2 | 17-04-2008 |
| | | ZA 200902012 A | 28-04-2010 |
| WO 2011088065 A1 | 21-07-2011 | US 2013145493 A1 | 06-06-2013 |
| | | WO 2011088065 A1 | 21-07-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008044150 A


### Non-patent literature cited in the description


- **BERG ; BERG.** *Biotechnol.,* 1983, vol. 1, 417-435
- **BERRY et al.** Kranz Anatomy and the C4 Pathway, In: Encyclopedia of Life Sciences. John Wiley & Sons, Ltd, 2008
- **BEVAN et al.** *Biotechnol.,* 1992, vol. 24, 367-70
- **CHRISTENSEN et al.** *Transgenic. Res.,* 1996, vol. 5, 213
- **DEPICKER et al.** *J. Mol. Appl. Genet.,* 1982, vol. 1, 561-73
- **DEPIGNY-THIS et al.** *Plant Molecular Biology,* 1992, vol. 20, 467-479
- **ENGELMANN et al.** *Plant Physiol,* 2008, vol. 246, 1773-1785
- **GOLDSBROUGH et al.** *Nature Biotechnology,* 1993, vol. 11, 1286-1292
- **HE et al.** *Maydica electronic publication,* 2014, vol. 59, 250-256
- Haworth's Food. Product Press, 2005, vol. 15, 213-257
- **HIREL et al.** *Physiol. Plant,* 2005, vol. 124, 178-188
- **ISHIDA et al.** *Nat. Biotechnol.,* 1996, vol. 14, 745-750
- **JOHN et al.** *Plant Physiol,* 2014, vol. 165, 62-75
- **KATAYAMA et al.** *Plant Mol. Biol.,* 2000, vol. 44, 99-106
- **KAY et al.** *Science,* 1987, vol. 236, 1299-1302
- **KOMARI et al.** *Plant J.,* 1996, vol. 10, 165-74
- **LEEGOOD et al.** *Journal of Experimental Botany,* 2008, vol. 59, 1663-1673
- **MARTIN et al.** *Plant Cell,* 2006, vol. 18, 3252-74
- **MCELROY et al.** *Plant Cell,* 1990, vol. 2, 163
- **MCELROY et al.** *Mol. Gen. Genet.,* 1991, vol. 231, 150-160
- **SAKAKIBARA et al.** *Plant Cell Physiol.,* 1992, vol. 33, 1193-1198
- **TATUSOVA ; MADDEN.** *FEMS Microbiol. Lett.,* 1999, vol. 174, 247-250
- **VERDAGUER et al.** *Plant Mol. Biol.,* 1996, vol. 6, 1129-39

SEQUENCE LISTING

<110> GENOPLANTE-VALOR

<120> IMPROVEMENT OF PLANT YIELD

<130> XRN/cc-F1516_42/EP

<160> 27

<170> PatentIn version 3.5

<210> 1
<211> 356
<212> PRT
<213> Zea mays

<400> 1

```
Met Ala Cys Leu Thr Asp Leu Val Asn Leu Asn Leu Ser Asp Asn Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Ser Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Arg Gly Asn Asn Ile Leu Val Met Cys Asp Cys Tyr Thr
                85                  90                  95

Pro Ala Gly Glu Pro Ile Pro Thr Asn Lys Arg Tyr Asn Ala Ala Lys
            100                 105                 110

Ile Phe Ser Ser Pro Glu Val Ala Ala Glu Glu Pro Trp Tyr Gly Ile
        115                 120                 125

Glu Gln Glu Tyr Thr Leu Leu Gln Lys Asp Thr Asn Trp Pro Leu Gly
    130                 135                 140

Trp Pro Ile Gly Gly Phe Pro Gly Pro Gln Gly Pro Tyr Tyr Cys Gly
145                 150                 155                 160

Ile Gly Ala Glu Lys Ser Phe Gly Arg Asp Ile Val Asp Ala His Tyr
                165                 170                 175
```

```
Lys Ala Cys Leu Tyr Ala Gly Ile Asn Ile Ser Gly Ile Asn Gly Glu
            180                 185                 190

Val Met Pro Gly Gln Trp Glu Phe Gln Val Gly Pro Ser Val Gly Ile
        195                 200                 205

Ser Ser Gly Asp Gln Val Trp Val Ala Arg Tyr Ile Leu Glu Arg Ile
    210                 215                 220

Thr Glu Ile Ala Gly Val Val Val Thr Phe Asp Pro Lys Pro Ile Pro
225                 230                 235                 240

Gly Asp Trp Asn Gly Ala Gly Ala His Thr Asn Tyr Ser Thr Glu Ser
                245                 250                 255

Met Arg Lys Glu Gly Gly Tyr Glu Val Ile Lys Ala Ala Ile Glu Lys
            260                 265                 270

Leu Lys Leu Arg His Arg Glu His Ile Ala Ala Tyr Gly Glu Gly Asn
        275                 280                 285

Glu Arg Arg Leu Thr Gly Arg His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Ile Trp Lys Pro
        355


<210>  2
<211>  5299
<212>  DNA
<213>  Artificial Sequence

<220>
<223>  proCsVMV-OsActin_intron-ZmGS1b-terminator ZmGS1b-terminator
       NOS-proZmRbcS-ZmGS1b-terminator ZmGS1b-terminator NOS

<400>  2
aaggtaatta tccaagatgt agcatcaaga atccaatgtt tacgggaaaa actatggaag      60

tattatgtga gctcagcaag aagcagatca atatgcggca catatgcaac ctatgttcaa     120

aaatgaagaa tgtacagata caagatccta tactgccaga atacgaagaa gaatacgtag     180
```

```
aaattgaaaa agaagaacca ggcgaagaaa agaatcttga agacgtaagc actgacgaca 240
acaatgaaaa gaagaagata aggtcggtga ttgtgaaaga gacatagagg acacatgtaa 300
ggtggaaaat gtaagggcgg aaagtaacct tatcacaaag gaatcttatc ccccactact 360
tatcctttta tatttttccg tgtcattttt gcccttgagt tttcctatat aaggaaccaa 420
gttcggcatt tgtgaaaaca agaaaaaatt tggtgtaagc tattttcttt gaagtactga 480
ggatacaact tcagagaaat ttgtccctcc cccctccccc tccgccgccg ccgcgccggt 540
aaccaccccg cccctctcct ctttctttct ccgttttttt tttccgtctc ggtctcgatc 600
tttggccttg gtagtttggg tgggcgagag gcggcttcgt gcgcgcccag atcggtgcgc 660
gggaggggcg ggatctcgcg gctggggctc tcgccggcgt ggatccggcc cggatctcgc 720
ggggaatggg gctctcggat gtagatctgc gatccgccgt tgttggggga gatgatgggg 780
ggtttaaaat ttccgccatg ctaaacaaga tcaggaagag gggaaaaggg cactatggtt 840
tatattttta tatatttctg ctgcttcgtc aggcttagat gtgctagatc tttctttctt 900
ctttttgtgg gtagaatttg aatccctcag cattgttcat cggtagtttt tcttttcatg 960
atttgtgaca aatgcagcct cgtgcggagc ttttttgtag gtagacgata agcttgatat 1020
cgaattcctg caggtcgact ctagaggatc caccccctgtc gcaccgcagc cgccggccat 1080
ggcctgcctc accgacctcg tcaacctcaa cctctcggac aacaccgaga agatcatcgc 1140
ggaatacata tggatcggtg gatctggcat ggatctcagg agcaaagcaa ggaccctctc 1200
cggcccggtg accgatccca gcaagctgcc caagtggaac tacgacggct ccagcacggg 1260
ccaggccccc ggcgaggaca gcgaggtcat cctgtacccg caggccatct tcaaggaccc 1320
attcaggagg ggcaacaaca tccttgtgat gtgcgattgc tacacccccag ccggcgagcc 1380
aatccccacc aacaagaggt acaacgccgc caagatcttc agcagccctg aggtcgccgc 1440
cgaggagccg tggtatggta ttgagcagga gtacaccctc ctccagaagg acaccaactg 1500
gccccttggg tggcccatcg gtggcttccc cggccctcag ggtccttact actgtggaat 1560
cggcgccgaa aagtcgttcg gccgcgacat cgtggacgcc cactacaagg cctgcttgta 1620
tgcgggcatc aacatcagtg gcatcaacgg ggaggtgatg ccagggcagt gggagttcca 1680
agtcgggcct tccgtgggta tatcttcagg cgaccaggtc tgggtcgctc gctacattct 1740
tgagaggatc acggagatcg ccggtgtggt ggtgacgttc gacccgaagc cgatcccggg 1800
cgactggaac ggcgccggcg cgcacaccaa ctacagcacg gagtcgatga ggaaggaggg 1860
cgggtacgag gtgatcaagg cggccatcga gaagctgaag ctgcggcaca gggagcacat 1920
cgcggcatac ggcgagggca acgagcgccg gctcaccggc aggcacgaga ccgccgacat 1980
caacacgttc agctggggcg tggccaaccg cggcgcgtcg gtgcgcgtgg gccgggagac 2040
```

ggagcagaac ggcaagggct acttcgagga ccgccgcccg gcgtccaaca tggacccta 2100
cgtggtcacc tccatgatcg ccgagaccac catcatctgg aagccctgag ggctaaggcg 2160
gccgttccgt cgcgttgcgt tgcgttgcag ggtccccgaa gcgattgcaa agccattgtt 2220
ccttctgttc cgtttgctta ttattatcat tattatcatc tagctagatc atccgggggt 2280
caggtcgtcg tggtgtgcca aaacagaaca cagaaagaag aagaaaaaaa aacaagacgt 2340
gtggcgttaa tgttatgtca tccaataatg gccgtaccac acttgtggta cctttcagta 2400
acttcgttgc ccggatcctc tagagtcgac ctgcagcccg ggggatccaa ttcccgatcg 2460
ttcaaacatt tggcaataaa gtttcttaag attgaatcct gttgccggtc ttgcgatgat 2520
tatcatataa tttctgttga attacgttaa gcatgtaata attaacatgt aatgcatgac 2580
gttatttatg agatgggttt ttatgattag agtcccgcaa ttatacattt aatacgcgat 2640
agaaaacaaa atatagcgcg caaactagga taaattatcg cgcgcggtgt catctatgtt 2700
actagtccct ttaatctggc gctagatctg catccgcggc ttgcaaagat aaatggcaca 2760
tttagtgtgt tattttgcaa tacctttcat agtagatatc cttaaatgca gttttaggca 2820
tgtttgggta attaaataac atttttagga ggagttttag atttaccttt ctttcgtgat 2880
gactgatgac agacgtgggg aattcaaatg caactctagc gaaagttcat atatttttca 2940
taaatagctg aggctggggt aattattttt tttgtagaaa aatagaatag gtggaatggt 3000
tggggaaggc gtaggcgctc gtggacgacg cccgataaaa gacaagaggc ggaattgcca 3060
tgaattcgag gtagctaagt aaggcgcata tatatgccaa aaaattctac tgtcactttc 3120
caatttcaat gcgctgccaa acaagccatc ctggaaactg acttgaattc agcccaattc 3180
tgtagatcca aacagggccg gcgtcagtgc ctcaggtgag agagcagcag acgatgcaaa 3240
gagccaaaac tgcaagcaga cgcagccgaa gccgaagccg aagcccaagc ccaaaactgt 3300
tttgtctttg cccagaaccg cgacgagcct aaactgcgct tcctcctatc tacaagtccc 3360
tggcacatca cgcatagtcc aacctaggcg cgcaggcgat aaggcgcgcc acggggacgc 3420
gacatgtggt ggcggacgcg atcaggatag ggccaggctg gccgggcgcg gccacgggag 3480
aacggtggcc actcgtccca catccgcttc gtcctgtcct gtactgcgtc ctgcccccaa 3540
cgagagccgg agccggccat cccgtcgcac actctccccc tctatatatg ccgtcggtgt 3600
gggggagcct actacaggac gacccaagca agcaagcaag cagcgagtac atacatacta 3660
ggcagccagg cagccaccat ggcctgcctc accgacctcg tcaacctcaa cctctcggac 3720
aacaccgaga agatcatcgc ggaatacata tggatcggtg gatctggcat ggatctcagg 3780
agcaaagcaa ggaccctctc cggcccggtg accgatccca gcaagctgcc caagtggaac 3840
tacgacggct ccagcacggg ccaggccccc ggcgaggaca gcgaggtcat cctgtacccg 3900
caggccatct tcaaggaccc attcaggagg ggcaacaaca tccttgtgat gtgcgattgc 3960

tacaccccag ccggcgagcc aatccccacc aacaagaggt acaacgccgc caagatcttc 4020

agcagccctg aggtcgccgc cgaggagccg tggtatggta ttgagcagga gtacaccctc 4080

ctccagaagg acaccaactg gccccttggg tggcccatcg gtggcttccc cggccctcag 4140

ggtccttact actgtggaat cggcgccgaa aagtcgttcg gccgcgacat cgtggacgcc 4200

cactacaagg cctgcttgta tgcgggcatc aacatcagtg gcatcaacgg ggaggtgatg 4260

ccagggcagt gggagttcca agtcgggcct tccgtgggta tatcttcagg cgaccaggtc 4320

tgggtcgctc gctacattct tgagaggatc acggagatcg ccggtgtggt ggtgacgttc 4380

gacccgaagc cgatcccggg cgactggaac ggcgccggcg cgcacaccaa ctacagcacg 4440

gagtcgatga ggaaggaggg cgggtacgag gtgatcaagg cggccatcga gaagctgaag 4500

ctgcggcaca gggagcacat cgcggcatac ggcgagggca acgagcgccg gctcaccggc 4560

aggcacgaga ccgccgacat caacacgttc agctggggcg tggccaaccg cggcgcgtcg 4620

gtgcgcgtgg gccgggagac ggagcagaac ggcaagggct acttcgagga ccgccgcccg 4680

gcgtccaaca tggaccccta cgtggtcacc tccatgatcg ccgagaccac catcatctgg 4740

aagccctgag ggctaaggcg gccgttccgt cgcgttgcgt tgcgttgcag ggtccccgaa 4800

gcgattgcaa agccattgtt ccttctgttc cgtttgctta ttattatcat tattatcatc 4860

tagctagatc atccgggggt caggtcgtcg tggtgtgcca aaacagaaca cagaaagaag 4920

aagaaaaaaa aacaagacgt gtggcgttaa tgttatgtca tccaataatg gccgtaccac 4980

acttgtggta cctttcagta acttcgttgc cggatcctct agagtcgacc tgcagcccgg 5040

gggatccaat tcccgatcgt tcaaacattt ggcaataaag tttcttaaga ttgaatcctg 5100

ttgccggtct tgcgatgatt atcatataat ttctgttgaa ttacgttaag catgtaataa 5160

ttaacatgta atgcatgacg ttatttatga gatgggtttt tatgattaga gtcccgcaat 5220

tatacattta atacgcgata gaaaacaaaa tatagcgcgc aaactaggat aaattatcgc 5280

gcgcggtgtc atctatgtt 5299

<210> 3
<211> 502
<212> DNA
<213> Cassava vein mosaic virus

<400> 3
aaggtaatta tccaagatgt agcatcaaga atccaatgtt tacgggaaaa actatggaag 60

tattatgtga gctcagcaag aagcagatca atatgcggca catatgcaac ctatgttcaa 120

aaatgaagaa tgtacagata caagatccta tactgccaga atacgaagaa gaatacgtag 180

aaattgaaaa agaagaacca ggcgaagaaa agaatcttga agacgtaagc actgacgaca 240

acaatgaaaa gaagaagata aggtcggtga ttgtgaaaga gacatagagg acacatgtaa 300

ggtggaaaat gtaagggcgg aaagtaacct tatcacaaag gaatcttatc ccccactact 360

tatcctttta tatttttccg tgtcattttt gcccttgagt tttcctatat aaggaaccaa 420

gttcggcatt tgtgaaaaca agaaaaaatt tggtgtaagc tattttcttt gaagtactga 480

ggatacaact tcagagaaat tt 502

<210> 4
<211> 462
<212> DNA
<213> Oryza sativa

<400> 4
gtaaccaccc cgcccctctc ctctttcttt ctccgttttt tttttccgtc tcggtctcga 60

tctttggcct tggtagtttg ggtgggcgag aggcggcttc gtgcgcgccc agatcggtgc 120

gcgggagggg cgggatctcg cggctggggc tctcgccggc gtggatccgg cccggatctc 180

gcggggaatg gggctctcgg atgtagatct gcgatccgcc gttgttgggg gagatgatgg 240

ggggtttaaa atttccgcca tgctaaacaa gatcaggaag aggggaaaag ggcactatgg 300

tttatatttt tatatatttc tgctgcttcg tcaggcttag atgtgctaga tctttctttc 360

ttctttttgt gggtagaatt tgaatccctc agcattgttc atcggtagtt tttcttttca 420

tgatttgtga caaatgcagc ctcgtgcgga gctttttttgt ag 462

<210> 5
<211> 1071
<212> DNA
<213> Zea mays

<400> 5
atggcctgcc tcaccgacct cgtcaacctc aacctctcgg acaacaccga gaagatcatc 60

gcggaataca tatggatcgg tggatctggc atggatctca ggagcaaagc aaggaccctc 120

tccggcccgg tgaccgatcc cagcaagctg cccaagtgga actacgacgg ctccagcacg 180

ggccaggccc ccggcgagga cagcgaggtc atcctgtacc cgcaggccat cttcaaggac 240

ccattcagga ggggcaacaa catccttgtg atgtgcgatt gctacacccc agccggcgag 300

ccaatcccca ccaacaagag gtacaacgcc gccaagatct tcagcagccc tgaggtcgcc 360

gccgaggagc cgtggtatgg tattgagcag gagtacaccc tcctccagaa ggacaccaac 420

tggccccttg ggtggcccat cggtggcttc cccggccctc agggtcctta ctactgtgga 480

atcggcgccg aaaagtcgtt cggccgcgac atcgtggacg cccactacaa ggcctgcttg 540

tatgcgggca tcaacatcag tggcatcaac ggggaggtga tgccagggca gtgggagttc 600

caagtcgggc cttccgtggg tatatcttca ggcgaccagg tctgggtcgc tcgctacatt 660

cttgagagga tcacggagat cgccggtgtg gtggtgacgt tcgacccgaa gccgatcccg 720

```
ggcgactgga acggcgccgg cgcgcacacc aactacagca cggagtcgat gaggaaggag      780

ggcgggtacg aggtgatcaa ggcggccatc gagaagctga agctgcggca cagggagcac      840

atcgcggcat acggcgaggg caacgagcgc cggctcaccg gcaggcacga gaccgccgac      900

atcaacacgt tcagctgggg cgtggccaac cgcggcgcgt cggtgcgcgt gggccgggag      960

acggagcaga acggcaaggg ctacttcgag gaccgccgcc cggcgtccaa catggacccc     1020

tacgtggtca cctccatgat cgccgagacc accatcatct ggaagccctg a              1071
```

```
<210>  6
<211>  252
<212>  DNA
<213>  Zea mays

<400>  6
gggctaaggc ggccgttccg tcgcgttgcg ttgcgttgca gggtccccga agcgattgca       60

aagccattgt tccttctgtt ccgtttgctt attattatca ttattatcat ctagctagat      120

catccggggg tcaggtcgtc gtggtgtgcc aaaacagaac acagaaagaa gaagaaaaaa      180

aaacaagacg tgtggcgtta atgttatgtc atccaataat ggccgtacca cacttgtggt      240

acctttcagt aa                                                          252
```

```
<210>  7
<211>  252
<212>  DNA
<213>  Agrobacterium tumefaciens

<400>  7
aattcccgat cgttcaaaca tttggcaata aagtttctta agattgaatc ctgttgccgg       60

tcttgcgatg attatcatat aatttctgtt gaattacgtt aagcatgtaa taattaacat      120

gtaatgcatg acgttattta tgagatgggt ttttatgatt agagtcccgc aattatacat      180

ttaatacgcg atagaaaaca aaatatagcg cgcaaactag gataaattat cgcgcgcggt      240

gtcatctatg tt                                                          252
```

```
<210>  8
<211>  970
<212>  DNA
<213>  Zea mays

<400>  8
tccctttaat ctggcgctag atctgcatcc gcggcttgca aagataaatg gcacatttag       60

tgtgttattt tgcaatacct ttcatagtag atatccttaa atgcagtttt aggcatgttt      120

gggtaattaa ataacatttt taggaggagt tttagattta cctttctttc gtgatgactg      180

atgacagacg tggggaattc aaatgcaact ctagcgaaag ttcatatatt tttcataaat      240

agctgaggct ggggtaatta tttttttgt agaaaaatag aataggtgga atggttgggg       300
```

aaggcgtagg cgctcgtgga cgacgcccga taaaagacaa gaggcggaat tgccatgaat 360

tcgaggtagc taagtaaggc gcatatatat gccaaaaaat tctactgtca ctttccaatt 420

tcaatgcgct gccaaacaag ccatcctgga aactgacttg aattcagccc aattctgtag 480

atccaaacag ggccggcgtc agtgcctcag gtgagagagc agcagacgat gcaaagagcc 540

aaaactgcaa gcagacgcag ccgaagccga agccgaagcc caagcccaaa actgttttgt 600

ctttgcccag aaccgcgacg agcctaaact gcgcttcctc ctatctacaa gtccctggca 660

catcacgcat agtccaacct aggcgcgcag gcgataaggc gcgccacggg gacgcgacat 720

gtggtggcgg acgcgatcag gatagggcca ggctggccgg gcgcggccac gggagaacgg 780

tggccactcg tcccacatcc gcttcgtcct gtcctgtact gcgtcctgcc cccaacgaga 840

gccggagccg gccatcccgt cgcacactct ccccctctat atatgccgtc ggtgtggggg 900

agcctactac aggacgaccc aagcaagcaa gcaagcagcg agtacataca tactaggcag 960

ccaggcagcc 970

<210> 9
<211> 356
<212> PRT
<213> Zea mays

<400> 9

Met Ala Cys Leu Thr Asp Leu Val Asn Leu Asn Leu Ser Asp Asn Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Ser Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Arg Gly Asn Asn Ile Leu Val Met Cys Asp Cys Tyr Thr
                85                  90                  95

Pro Ala Gly Glu Pro Ile Pro Thr Asn Lys Arg Tyr Asp Ala Ala Lys
            100                 105                 110

Ile Phe Ser Ser Pro Glu Val Ala Ala Glu Glu Pro Trp Tyr Gly Ile
        115                 120                 125

```
Glu Gln Glu Tyr Thr Leu Leu Gln Lys Asp Thr Asn Trp Pro Leu Gly
    130                 135                 140

Trp Pro Ile Gly Gly Phe Pro Gly Pro Gln Gly Pro Tyr Tyr Cys Gly
145                 150                 155                 160

Ile Gly Ala Glu Lys Ser Phe Gly Arg Asp Ile Val Asp Ala His Tyr
                165                 170                 175

Lys Ala Cys Leu Tyr Ala Gly Ile Asn Ile Ser Gly Ile Asn Gly Glu
            180                 185                 190

Val Met Pro Gly Gln Trp Glu Phe Gln Val Gly Pro Ser Val Gly Ile
        195                 200                 205

Ser Ser Gly Asp Gln Val Trp Val Ala Arg Tyr Ile Leu Glu Arg Ile
    210                 215                 220

Thr Glu Ile Ala Gly Val Val Val Thr Phe Asp Pro Lys Pro Ile Pro
225                 230                 235                 240

Gly Asp Trp Asn Ala Ala Gly Ala His Thr Asn Tyr Ser Thr Glu Ser
                245                 250                 255

Met Arg Lys Glu Gly Gly Tyr Glu Val Ile Lys Ala Ala Ile Glu Lys
            260                 265                 270

Leu Lys Leu Arg His Arg Glu His Ile Ala Ala Tyr Gly Glu Gly Asn
        275                 280                 285

Glu Arg Arg Leu Thr Gly Arg His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Ile Trp Lys Pro
        355
```

<210> 10
<211> 356

<212> PRT
<213> Zea mays

<400> 10

```
Met Ala Cys Leu Thr Asp Leu Val Asn Leu Asn Leu Ser Asp Thr Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Pro Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Arg Gly Asn Asn Ile Leu Val Met Cys Asp Cys Tyr Thr
                85                  90                  95

Pro Ala Gly Glu Pro Ile Pro Thr Asn Lys Arg Tyr Ser Ala Ala Lys
            100                 105                 110

Ile Phe Ser Ser Pro Glu Val Ala Ala Glu Glu Pro Trp Tyr Gly Ile
        115                 120                 125

Glu Gln Glu Tyr Thr Leu Leu Gln Lys Asp Thr Asn Trp Pro Leu Gly
    130                 135                 140

Trp Pro Ile Gly Gly Phe Pro Gly Pro Gln Gly Pro Tyr Tyr Cys Gly
145                 150                 155                 160

Ile Gly Ala Glu Lys Ser Phe Gly Arg Asp Ile Val Asp Ala His Tyr
                165                 170                 175

Lys Ala Cys Leu Tyr Ala Gly Ile Asn Ile Ser Gly Ile Asn Gly Glu
            180                 185                 190

Val Met Pro Gly Gln Trp Glu Phe Gln Val Gly Pro Thr Val Gly Ile
        195                 200                 205

Ser Ser Gly Asp Gln Val Trp Val Ala Arg Tyr Ile Leu Glu Arg Ile
    210                 215                 220

Thr Glu Ile Ala Gly Val Val Val Thr Phe Asp Pro Lys Pro Ile Pro
225                 230                 235                 240
```

```
Gly Asp Trp Asn Gly Ala Gly Ala His Thr Asn Tyr Ser Thr Glu Ser
                245                 250                 255

Met Arg Lys Glu Gly Gly Tyr Glu Val Ile Lys Ala Ala Ile Glu Lys
            260                 265                 270

Leu Lys Leu Arg His Lys Glu His Ile Ala Ala Tyr Gly Glu Gly Asn
        275                 280                 285

Glu Arg Arg Leu Thr Gly Arg His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Ile Trp Lys Pro
        355


<210>  11
<211>  356
<212>  PRT
<213>  Zea mays

<400>  11

Met Ala Cys Leu Thr Asp Leu Val Asn Leu Asn Leu Ser Asp Thr Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Pro Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Arg Gly Asn Asn Ile Leu Val Met Cys Asp Cys Tyr Thr
                85                  90                  95
```

Pro Ala Gly Glu Pro Ile Pro Thr Asn Lys Arg Tyr Ser Ala Ala Lys
100 105 110

Ile Phe Ser Ser Leu Glu Val Ala Ala Glu Glu Pro Trp Tyr Gly Ile
115 120 125

Glu Gln Glu Tyr Thr Leu Leu Gln Lys Asp Thr Asn Trp Pro Leu Gly
130 135 140

Trp Pro Ile Gly Gly Phe Pro Gly Pro Gln Gly Pro Tyr Tyr Cys Gly
145 150 155 160

Ile Gly Ala Glu Lys Ser Phe Gly Arg Asp Ile Val Asp Ala His Tyr
165 170 175

Lys Ala Cys Leu Tyr Ala Gly Ile Asn Ile Ser Gly Ile Asn Gly Glu
180 185 190

Val Met Pro Gly Gln Trp Glu Phe Gln Val Gly Pro Ser Val Gly Ile
195 200 205

Ser Ser Gly Asp Gln Val Trp Val Ala Arg Tyr Ile Leu Glu Arg Ile
210 215 220

Thr Glu Ile Ala Gly Val Val Val Thr Phe Asp Pro Lys Pro Ile Pro
225 230 235 240

Gly Asp Trp Asn Gly Ala Gly Ala His Thr Asn Tyr Ser Thr Glu Ser
245 250 255

Met Arg Lys Glu Gly Gly Tyr Glu Val Ile Lys Ala Ala Ile Glu Lys
260 265 270

Leu Lys Leu Arg His Lys Glu His Ile Ala Ala Tyr Gly Glu Gly Asn
275 280 285

Glu Arg Arg Leu Thr Gly Arg His Glu Thr Ala Asp Ile Asn Thr Phe
290 295 300

Ser Trp Gly Val Ala Asn Arg Gly Ala Ser Val Arg Val Gly Arg Glu
305 310 315 320

Thr Glu Gln Asn Gly Lys Gly Tyr Phe Glu Asp Arg Arg Pro Ala Ser
325 330 335

Asn Met Asp Pro Tyr Val Val Thr Ser Met Ile Ala Glu Thr Thr Ile

340 345 350

Val Trp Lys Pro
355

<210> 12
<211> 356
<212> PRT
<213> Zea mays

<400> 12

Met Ala Cys Leu Thr Asp Leu Val Asn Leu Asn Leu Ser Asp Thr Thr
1 5 10 15

Glu Lys Ile Ile Ala Glu Tyr Ile Trp Ile Gly Gly Ser Gly Met Asp
20 25 30

Leu Arg Ser Lys Ala Arg Thr Leu Pro Gly Pro Val Thr Asp Pro Ser
35 40 45

Lys Leu Pro Lys Trp Asn Tyr Asp Gly Ser Ser Thr Gly Gln Ala Pro
50 55 60

Gly Glu Tyr Ser Glu Val Ile Leu Tyr Pro Gln Ala Ile Phe Lys Asp
65 70 75 80

Pro Phe Arg Arg Gly Asn Asn Ile Leu Val Met Cys Asp Cys Tyr Thr
85 90 95

Pro Ala Gly Glu Pro Ile Pro Thr Asn Lys Arg Tyr Ser Ala Ala Lys
100 105 110

Ile Phe Ser Ser Pro Glu Leu Ala Ala Glu Glu Pro Trp Tyr Gly Ile
115 120 125

Glu Gln Glu Tyr Thr Leu Leu Gln Lys Asp Thr Asn Trp Pro Leu Gly
130 135 140

Trp Pro Ile Gly Gly Phe Pro Gly Pro Gln Gly Pro Tyr Tyr Cys Gly
145 150 155 160

Ile Gly Ala Glu Lys Ser Phe Gly Arg Asp Ile Val Asp Ala His Tyr
165 170 175

Lys Ala Cys Leu Tyr Ala Gly Ile Asn Ile Ser Gly Ile Asn Gly Glu
180 185 190

Val Met Pro Gly Gln Trp Glu Phe Gln Val Gly Pro Ser Val Gly Ile

195 200 205

Ser Ser Gly Asp Gln Val Trp Val Ala Arg Tyr Ile Leu Glu Arg Ile
210 215 220

Thr Glu Ile Ala Gly Val Val Val Thr Phe Asp Pro Lys Pro Ile Pro
225 230 235 240

Gly Asp Trp Asn Gly Ala Gly Ala His Thr Asn Tyr Ser Thr Glu Ser
245 250 255

Met Arg Lys Glu Gly Gly Tyr Glu Val Ile Lys Ala Ala Ile Glu Lys
260 265 270

Leu Lys Leu Arg His Lys Glu His Ile Ala Ala Tyr Gly Glu Gly Asn
275 280 285

Glu Arg Arg Leu Thr Gly Arg His Glu Thr Ala Asp Ile Asn Thr Phe
290 295 300

Ser Trp Gly Val Ala Asn Arg Gly Ala Ser Val Arg Val Gly Arg Glu
305 310 315 320

Thr Glu Gln Asn Gly Lys Gly Tyr Phe Glu Asp Arg Arg Pro Ala Ser
325 330 335

Asn Met Asp Pro Tyr Val Val Thr Ser Met Ile Ala Glu Thr Thr Ile
340 345 350

Val Trp Lys Pro
355

<210> 13
<211> 356
<212> PRT
<213> Zea mays

<400> 13

Met Ala Cys Leu Thr Asp Leu Val Asn Leu Asn Leu Ser Asp Thr Thr
1 5 10 15

Glu Lys Phe Ile Ala Glu Tyr Ile Trp Ile Gly Gly Ser Gly Met Asp
20 25 30

Leu Arg Ser Lys Ala Arg Thr Leu Pro Gly Pro Val Thr Asp Pro Ser
35 40 45

Lys Leu Pro Lys Trp Asn Tyr Asp Gly Ser Ser Thr Gly Gln Ala Pro

50 55 60

Gly Glu Asp Ser Glu Val Ile Leu Tyr Pro Gln Ala Ile Phe Lys Asp
65 70 75 80

Pro Phe Arg Arg Gly Asn Asn Ile Leu Val Met Cys Asp Cys Tyr Thr
85 90 95

Pro Ala Gly Glu Pro Ile Pro Thr Asn Lys Arg Tyr Ser Ala Ala Lys
100 105 110

Ile Phe Ser Ser Pro Glu Val Ala Ala Glu Glu Pro Trp Tyr Gly Ile
115 120 125

Glu Gln Glu Tyr Thr Leu Leu Gln Lys Asp Thr Asn Trp Pro Leu Gly
130 135 140

Trp Pro Ile Gly Gly Phe Pro Gly Pro Gln Gly Pro Tyr Tyr Cys Gly
145 150 155 160

Ile Gly Ala Glu Lys Ser Phe Gly Arg Asp Thr Val Asp Ala His Tyr
165 170 175

Lys Ala Cys Leu Tyr Ala Gly Ile Asn Ile Ser Gly Ile Asn Gly Glu
180 185 190

Val Met Pro Gly Gln Trp Glu Phe Gln Val Gly Pro Ser Val Gly Ile
195 200 205

Ser Ser Gly Asp Gln Val Trp Val Ala Arg Tyr Val Leu Glu Arg Ile
210 215 220

Thr Glu Ile Ala Gly Val Val Val Thr Phe Asp Pro Lys Pro Ile Pro
225 230 235 240

Gly Gly Trp Asn Gly Ala Gly Ala His Thr Asn Tyr Ser Thr Glu Ser
245 250 255

Met Arg Lys Glu Gly Gly Tyr Glu Val Ile Lys Ala Ala Ile Glu Lys
260 265 270

Leu Lys Leu Arg His Lys Glu His Ile Ala Ala Tyr Gly Glu Gly Asn
275 280 285

Glu Arg Arg Leu Thr Gly Arg His Glu Thr Ala Asp Ile Asn Thr Phe
290 295 300

```
Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Val Trp Lys Pro
        355


<210>  14
<211>  355
<212>  PRT
<213>  Zea mays

<400>  14

Met Ala Cys Leu Thr Asp Leu Val Asn Leu Asn Leu Ser Asp Thr Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Pro Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Arg Gly Asn Asn Ile Leu Val Met Cys Asp Cys Tyr Thr
                85                  90                  95

Pro Ala Gly Glu Pro Ile Pro Thr Asn Lys Arg Tyr Ser Ala Ala Lys
            100                 105                 110

Ile Phe Ser Ser Pro Glu Val Ala Ala Glu Glu Pro Trp Tyr Gly Ile
        115                 120                 125

Glu Gln Glu Tyr Thr Leu Leu Gln Lys Asp Thr Asn Trp Pro Leu Gly
    130                 135                 140

Trp Pro Ile Gly Gly Phe Pro Gly Pro Gln Gly Pro Tyr Tyr Cys Gly
145                 150                 155                 160
```

```
Ile Gly Ala Glu Lys Ser Phe Gly Arg Asp Ile Val Asp Ala His Tyr
                165                 170                 175

Lys Ala Cys Leu Tyr Ala Gly Ile Asn Ile Ser Gly Ile Asn Gly Glu
            180                 185                 190

Val Met Pro Gly Gln Trp Glu Phe Gln Val Gly Pro Ser Val Gly Ile
        195                 200                 205

Ser Ser Gly Asp Gln Val Trp Val Ala Arg Tyr Ile Leu Glu Arg Ile
    210                 215                 220

Thr Glu Ile Ala Gly Val Val Val Thr Phe Asp Pro Lys Pro Ile Pro
225                 230                 235                 240

Gly Asp Trp Asn Gly Ala Gly Ala His Thr Asn Tyr Ser Thr Glu Ser
                245                 250                 255

Met Arg Lys Glu Gly Gly Tyr Glu Val Ile Lys Ala Ala Ile Glu Lys
            260                 265                 270

Leu Lys Leu Arg His Lys Glu His Ile Ala Ala Tyr Gly Glu Gly Asn
        275                 280                 285

Glu Arg Arg Leu Thr Gly Arg His Glu Thr Ala Asp Ile Asn Thr Phe
    290                 295                 300

Ser Trp Gly Val Ala Asn Arg Gly Ala Ser Val Ala Val Gly Gln Thr
305                 310                 315                 320

Glu Gln Asn Gly Lys Gly Tyr Phe Glu Asp Arg Arg Pro Ala Ser Asn
                325                 330                 335

Met Asp Pro Tyr Val Val Thr Ser Met Ile Ala Glu Thr Thr Ile Val
            340                 345                 350

Trp Lys Pro
        355


<210>  15
<211>  356
<212>  PRT
<213>  Setaria italica

<400>  15

Met Ala Cys Leu Thr Asp Leu Val Asn Leu Asn Leu Ser Asp Thr Thr
1               5                   10                  15
```

```
Glu Lys Ile Ile Ala Glu Tyr Ile Trp Ile Gly Gly Ser Gly Met Asp
            20                  25                  30

Leu Arg Ser Lys Ala Arg Thr Leu Ser Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Lys Gly Asn Asn Ile Leu Val Met Cys Asp Cys Tyr Thr
                85                  90                  95

Pro Ala Gly Glu Pro Ile Pro Thr Asn Lys Arg His Asn Ala Ala Lys
            100                 105                 110

Ile Phe Ser Asn Pro Glu Val Ser Ala Glu Glu Pro Trp Tyr Gly Ile
        115                 120                 125

Glu Gln Glu Tyr Thr Leu Leu Gln Lys Asp Thr Asn Trp Pro Leu Gly
    130                 135                 140

Trp Pro Val Gly Gly Phe Pro Gly Pro Gln Gly Pro Tyr Tyr Cys Gly
145                 150                 155                 160

Ile Gly Ala Asp Lys Ser Phe Gly Arg Asp Ile Val Asp Ser His Tyr
                165                 170                 175

Lys Ala Cys Leu Tyr Ala Gly Ile Asn Ile Ser Gly Ile Asn Gly Glu
            180                 185                 190

Val Met Pro Gly Gln Trp Glu Phe Gln Val Gly Pro Ser Val Gly Ile
        195                 200                 205

Ser Ala Gly Asp Gln Val Trp Val Ala Arg Tyr Ile Leu Glu Arg Ile
    210                 215                 220

Thr Glu Ile Ala Gly Val Val Val Thr Phe Asp Pro Lys Pro Ile Pro
225                 230                 235                 240

Gly Asp Trp Asn Gly Ala Gly Ala His Thr Asn Tyr Ser Thr Lys Ser
                245                 250                 255

Met Arg Asn Asp Gly Gly Tyr Glu Val Ile Lys Ala Ala Ile Glu Lys
            260                 265                 270
```

Leu Lys Leu Arg His Lys Glu His Ile Ala Ala Tyr Gly Glu Gly Asn
        275                 280                 285

Glu Arg Arg Leu Thr Gly Lys His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Ile Trp Lys Pro
        355

<210> 16
<211> 356
<212> PRT
<213> Saccharum officinarum

<400> 16

Met Ala Ser Leu Thr Asp Leu Val Asn Leu Ser Leu Ser Asp Thr Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Ser Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Arg Gly Asn Asn Ile Leu Val Met Cys Asp Cys Tyr Thr
                85                  90                  95

Pro Ala Gly Glu Pro Ile Pro Thr Asn Lys Arg His Asn Ala Ala Lys
            100                 105                 110

Ile Phe Ser Asn Pro Glu Val Ala Ala Glu Glu Pro Trp Tyr Gly Ile
        115                 120                 125

```
Glu Gln Glu Tyr Thr Leu Leu Gln Lys Asp Thr Asn Trp Pro Leu Gly
    130                 135                 140

Trp Pro Ile Gly Gly Phe Pro Gly Pro Gln Gly Pro Tyr Tyr Cys Gly
145                 150                 155                 160

Ile Gly Ala Asp Lys Ser Phe Gly Arg Asp Ile Val Asp Ala His Tyr
                165                 170                 175

Lys Ala Cys Leu Tyr Ala Gly Ile Asn Ile Ser Gly Ile Asn Gly Glu
            180                 185                 190

Val Met Pro Gly Gln Trp Glu Phe Gln Val Gly Pro Ser Val Gly Ile
        195                 200                 205

Ser Ser Gly Asp Gln Val Trp Val Ala Arg Tyr Ile Leu Glu Arg Ile
    210                 215                 220

Thr Glu Ile Ala Gly Val Val Leu Thr Phe Asp Pro Lys Pro Ile Pro
225                 230                 235                 240

Gly Asp Trp Asn Gly Ala Gly Ala His Thr Asn Tyr Ser Thr Lys Ser
                245                 250                 255

Met Arg Asn Glu Gly Gly Tyr Glu Val Ile Lys Ala Ala Ile Glu Lys
            260                 265                 270

Leu Lys Leu Arg His Lys Glu His Ile Ala Ala Tyr Gly Glu Gly Asn
        275                 280                 285

Glu Arg Arg Leu Thr Gly Arg His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Asp Thr Thr Ile
            340                 345                 350

Leu Trp Lys Pro
        355

<210>  17
<211>  356
```

<212> PRT
<213> Oryza brachyantha

<400> 17

Met Ala Ser Leu Thr Asp Leu Val Asn Leu Asn Leu Ser Asp Thr Thr
1 5 10 15

Glu Lys Ile Ile Ala Glu Tyr Ile Trp Ile Gly Gly Ser Gly Met Asp
20 25 30

Leu Arg Ser Lys Ala Arg Thr Ile Ser Gly Pro Val Thr Asp Pro Ser
35 40 45

Lys Leu Pro Lys Trp Asn Tyr Asp Gly Ser Ser Thr Gly Gln Ala Pro
50 55 60

Gly Glu Asp Ser Glu Val Ile Leu Tyr Pro Gln Ala Ile Phe Lys Asp
65 70 75 80

Pro Phe Arg Arg Gly Asn Asn Ile Leu Val Met Cys Asp Cys Tyr Thr
85 90 95

Pro Ala Gly Glu Pro Ile Pro Thr Asn Lys Arg Tyr Asn Ala Ala Lys
100 105 110

Ile Phe Ser Asn Pro Asp Val Ala Ala Glu Glu Pro Trp Tyr Gly Ile
115 120 125

Glu Gln Glu Tyr Thr Leu Leu Gln Lys Asp Ile Asn Trp Pro Leu Gly
130 135 140

Trp Pro Val Gly Gly Phe Pro Gly Pro Gln Gly Pro Tyr Tyr Cys Gly
145 150 155 160

Ile Gly Ala Asp Lys Ser Phe Gly Arg Asp Ile Val Asp Ser His Tyr
165 170 175

Lys Ala Cys Leu Tyr Ala Gly Ile Asn Ile Ser Gly Ile Asn Gly Glu
180 185 190

Val Met Pro Gly Gln Trp Glu Phe Gln Val Gly Pro Ser Val Gly Ile
195 200 205

Ser Ser Gly Asp Gln Leu Trp Val Ala Arg Tyr Ile Leu Glu Arg Ile
210 215 220

Thr Glu Ile Ala Gly Val Val Val Thr Phe Asp Pro Lys Pro Ile Pro
225 230 235 240

Gly Asp Trp Asn Gly Ala Gly Ala His Cys Asn Tyr Ser Thr Lys Ser
245 250 255

Met Arg Asn Asp Gly Gly Tyr Glu Ile Ile Lys Ser Ala Ile Glu Lys
260 265 270

Leu Lys Leu Arg His Lys Glu His Ile Ala Ala Tyr Gly Glu Gly Asn
275 280 285

Glu Arg Arg Leu Thr Gly Arg His Glu Thr Ala Asp Ile Asn Thr Phe
290 295 300

Ser Trp Gly Val Ala Asn Arg Gly Ala Ser Val Arg Val Gly Arg Glu
305 310 315 320

Thr Glu Gln Asn Gly Lys Gly Tyr Phe Glu Asp Arg Arg Pro Ala Ser
325 330 335

Asn Met Asp Pro Tyr Val Val Thr Ser Met Ile Ala Glu Thr Thr Ile
340 345 350

Ile Trp Lys Pro
355

<210> 18
<211> 356
<212> PRT
<213> Oryza sativa

<400> 18

Met Ala Ser Leu Thr Asp Leu Val Asn Leu Asn Leu Ser Asp Thr Thr
1 5 10 15

Glu Lys Ile Ile Ala Glu Tyr Ile Trp Ile Gly Gly Ser Gly Met Asp
20 25 30

Leu Arg Ser Lys Ala Arg Thr Leu Ser Gly Pro Val Thr Asp Pro Ser
35 40 45

Lys Leu Pro Lys Trp Asn Tyr Asp Gly Ser Ser Thr Gly Gln Ala Pro
50 55 60

Gly Glu Asp Ser Glu Val Ile Leu Tyr Pro Gln Ala Ile Phe Lys Asp
65 70 75 80

Pro Phe Arg Lys Gly Asn Asn Ile Leu Val Met Cys Asp Cys Tyr Thr
85 90 95

```
Pro Ala Gly Glu Pro Ile Pro Thr Asn Lys Arg His Asn Ala Ala Lys
            100                 105                 110

Ile Phe Ser Ser Pro Glu Val Ala Ser Glu Glu Pro Trp Tyr Gly Ile
        115                 120                 125

Glu Gln Glu Tyr Thr Leu Leu Gln Lys Asp Ile Asn Trp Pro Leu Gly
    130                 135                 140

Trp Pro Val Gly Gly Phe Pro Gly Pro Gln Gly Pro Tyr Tyr Cys Gly
145                 150                 155                 160

Ile Gly Ala Asp Lys Ser Phe Gly Arg Asp Ile Val Asp Ser His Tyr
                165                 170                 175

Lys Ala Cys Leu Tyr Ala Gly Ile Asn Ile Ser Gly Ile Asn Gly Glu
            180                 185                 190

Val Met Pro Gly Gln Trp Glu Phe Gln Val Gly Pro Ser Val Gly Ile
        195                 200                 205

Ser Ala Gly Asp Gln Val Trp Val Ala Arg Tyr Ile Leu Glu Arg Ile
    210                 215                 220

Thr Glu Ile Ala Gly Val Val Val Ser Phe Asp Pro Lys Pro Ile Pro
225                 230                 235                 240

Gly Asp Trp Asn Gly Ala Gly Ala His Thr Asn Tyr Ser Thr Lys Ser
                245                 250                 255

Met Arg Asn Asp Gly Gly Tyr Glu Ile Ile Lys Ser Ala Ile Glu Lys
            260                 265                 270

Leu Lys Leu Arg His Lys Glu His Ile Ser Ala Tyr Gly Glu Gly Asn
        275                 280                 285

Glu Arg Arg Leu Thr Gly Arg His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Ile Val Thr Ser Met Ile Ala Glu Thr Thr Ile
```

340 345 350

Ile Trp Lys Pro
355

<210> 19
<211> 356
<212> PRT
<213> Oryza glaberrima

<400> 19

Met Ala Ser Leu Thr Asp Leu Val Asn Leu Asn Leu Ser Asp Thr Thr
1 5 10 15

Glu Lys Ile Ile Ala Glu Tyr Ile Trp Ile Gly Gly Ser Gly Met Asp
20 25 30

Leu Arg Ser Lys Ala Arg Thr Leu Ser Gly Pro Val Thr Asp Pro Ile
35 40 45

Lys Leu Pro Lys Trp Asn Tyr Asp Gly Ser Ser Thr Gly Gln Ala Pro
50 55 60

Gly Glu Asp Ser Glu Val Ile Leu Tyr Pro Gln Ala Ile Phe Lys Asp
65 70 75 80

Pro Phe Arg Lys Gly Asn Asn Ile Leu Val Met Cys Asp Cys Tyr Thr
85 90 95

Pro Ala Gly Glu Pro Ile Pro Thr Asn Lys Arg His Asn Ala Ala Lys
100 105 110

Ile Phe Ser Ser Pro Glu Val Ala Ser Glu Glu Pro Trp Tyr Gly Ile
115 120 125

Glu Gln Glu Tyr Thr Leu Leu Gln Lys Asp Ile Asn Trp Pro Leu Gly
130 135 140

Trp Pro Val Gly Gly Phe Pro Gly Pro Gln Gly Pro Tyr Tyr Cys Gly
145 150 155 160

Ile Gly Ala Asp Lys Ser Phe Gly Arg Asp Ile Val Asp Ser His Tyr
165 170 175

Lys Ala Cys Leu Tyr Ala Gly Ile Asn Ile Ser Gly Ile Asn Gly Glu
180 185 190

Val Met Pro Gly Gln Trp Glu Phe Gln Val Gly Pro Ser Val Gly Ile

```
        195                 200                 205

Ser Ala Gly Asp Gln Val Trp Val Ala Arg Tyr Ile Leu Glu Arg Ile
    210                 215                 220

Thr Glu Ile Ala Gly Val Val Val Ser Phe Asp Pro Lys Pro Ile Pro
225                 230                 235                 240

Gly Asp Trp Asn Gly Ala Gly Ala His Thr Asn Tyr Ser Thr Lys Ser
                245                 250                 255

Met Arg Asn Asp Gly Gly Tyr Glu Ile Ile Lys Ser Ala Ile Glu Lys
            260                 265                 270

Leu Lys Leu Arg His Lys Glu His Ile Ser Ala Tyr Gly Glu Gly Asn
        275                 280                 285

Glu Arg Arg Leu Thr Gly Arg His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Ile Trp Lys Pro
        355

<210>  20
<211>  356
<212>  PRT
<213>  Brachypodium distachyon

<400>  20

Met Ala Leu Leu Thr Asp Leu Leu Asn Leu Asp Leu Ser Asp Ser Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Ser Gly Pro Val Thr Asp Pro Ser
        35                  40                  45

Lys Leu Pro Lys Trp Asn Tyr Asp Gly Ser Ser Thr Gly Gln Ala Pro
```

50 55 60

Gly Glu Asp Ser Glu Val Ile Leu Tyr Pro Gln Ala Ile Phe Lys Asp
65 70 75 80

Pro Phe Arg Arg Gly Asn Asn Ile Leu Val Met Cys Asp Cys Tyr Thr
85 90 95

Pro Ala Gly Glu Pro Ile Pro Thr Asn Lys Arg His Ala Ala Ala Lys
100 105 110

Ile Phe Ser Asn Pro Ala Val Ser Ala Glu Glu Pro Trp Tyr Gly Ile
115 120 125

Glu Gln Glu Tyr Thr Leu Leu Gln Lys Asp Ile Asn Trp Pro Leu Gly
130 135 140

Trp Pro Val Gly Gly Phe Pro Gly Pro Gln Gly Pro Tyr Tyr Cys Ser
145 150 155 160

Ile Gly Ala Asp Lys Ser Phe Gly Arg Asp Ile Val Asp Ser His Tyr
165 170 175

Lys Ala Cys Leu Tyr Ala Gly Ile Asn Ile Ser Gly Ile Asn Gly Glu
180 185 190

Val Met Pro Gly Gln Trp Glu Phe Gln Val Gly Pro Ser Val Gly Ile
195 200 205

Ser Ala Gly Asp Gln Val Trp Val Ala Arg Tyr Leu Leu Glu Arg Ile
210 215 220

Thr Glu Ile Ala Gly Val Val Val Ser Phe Asp Pro Lys Pro Ile Pro
225 230 235 240

Gly Asp Trp Asn Gly Ala Gly Ala His Thr Asn Tyr Ser Thr Glu Ser
245 250 255

Met Arg Lys Asp Gly Gly Phe Gln Val Ile Val Ala Ala Val Glu Lys
260 265 270

Leu Lys Leu Arg His Lys Glu His Ile Ala Ala Tyr Gly Glu Gly Asn
275 280 285

Glu Arg Arg Leu Thr Gly Lys His Glu Thr Ala Asp Ile His Thr Phe
290 295 300

Ser Trp Gly Val Ala Asn Arg Gly Ala Ser Val Arg Val Gly Arg Glu
305 310 315 320

Thr Glu Gln Asn Gly Lys Gly Tyr Phe Glu Asp Arg Arg Pro Ala Ser
325 330 335

Asn Met Asp Pro Tyr Val Val Thr Ala Met Ile Ala Asp Thr Thr Ile
340 345 350

Leu Trp Lys Pro
355

<210> 21
<211> 356
<212> PRT
<213> Hordeum vulgare

<400> 21

Met Ala Leu Leu Thr Asp Leu Leu Asn Leu Asp Leu Ser Gly Ser Thr
1 5 10 15

Glu Lys Ile Ile Ala Glu Tyr Ile Trp Ile Gly Gly Ser Gly Met Asp
20 25 30

Leu Arg Ser Lys Ala Arg Thr Leu Pro Gly Pro Val Thr Asp Pro Ser
35 40 45

Lys Leu Pro Lys Trp Asn Tyr Asp Gly Ser Ser Thr Gly Gln Ala Pro
50 55 60

Gly Glu Asp Ser Glu Val Ile Leu Tyr Pro Gln Ala Ile Phe Lys Asp
65 70 75 80

Pro Phe Arg Lys Gly Asn Asn Ile Leu Val Met Cys Asp Cys Tyr Thr
85 90 95

Pro Ala Gly Glu Pro Ile Pro Thr Asn Lys Arg Tyr Asn Ala Ala Lys
100 105 110

Ile Phe Ser Asn Pro Asp Val Ala Lys Glu Glu Pro Trp Tyr Gly Ile
115 120 125

Glu Gln Glu Tyr Thr Leu Leu Gln Lys Asp Ile Asn Trp Pro Leu Gly
130 135 140

Trp Pro Val Gly Gly Phe Pro Gly Pro Gln Gly Pro Tyr Tyr Cys Gly
145 150 155 160

```
Ile Gly Ala Asp Lys Ser Phe Gly Arg Asp Ile Val Asp Ser His Tyr
                165                 170                 175

Lys Ala Cys Leu Phe Ala Gly Val Asn Ile Ser Gly Ile Asn Gly Glu
            180                 185                 190

Val Met Pro Gly Gln Trp Glu Phe Gln Val Gly Pro Thr Val Gly Ile
        195                 200                 205

Ser Ala Gly Asp Gln Val Trp Val Ala Arg Tyr Ile Leu Glu Arg Ile
    210                 215                 220

Thr Glu Ile Ala Gly Val Val Val Thr Phe Asp Pro Lys Pro Ile Pro
225                 230                 235                 240

Gly Asp Trp Asn Gly Ala Gly Ala His Thr Asn Tyr Ser Thr Glu Ser
                245                 250                 255

Met Arg Asn Asp Gly Gly Phe Lys Val Ile Val Asp Ala Val Glu Lys
            260                 265                 270

Leu Lys Leu Lys His Lys Glu His Ile Ala Ala Tyr Gly Glu Gly Asn
        275                 280                 285

Glu Arg Arg Leu Thr Gly Lys His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Leu Trp Lys Pro
        355
```

<210> 22
<211> 356
<212> PRT
<213> Triticum aestivum

<400> 22

```
Met Ala Leu Leu Thr Asp Leu Leu Asn Leu Asp Leu Thr Asp Ser Thr
1               5                   10                  15
```

```
Glu Lys Ile Ile Ala Glu Tyr Ile Trp Ile Gly Gly Ser Gly Met Asp
            20                  25                  30

Leu Arg Ser Lys Ala Arg Thr Leu Pro Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Lys Gly Asn Asn Ile Leu Val Met Cys Asp Cys Tyr Thr
                85                  90                  95

Pro Ala Gly Val Pro Ile Pro Thr Asn Lys Arg Tyr Asn Ala Ala Lys
            100                 105                 110

Ile Phe Ser Asn Pro Asp Val Ala Lys Glu Glu Pro Trp Tyr Gly Ile
        115                 120                 125

Glu Gln Glu Tyr Thr Leu Leu Gln Lys Asp Ile Asn Trp Pro Leu Gly
    130                 135                 140

Trp Pro Val Gly Gly Phe Pro Gly Pro Gln Gly Pro Tyr Tyr Cys Ser
145                 150                 155                 160

Ile Gly Ala Asp Lys Ser Phe Gly Arg Asp Ile Val Asp Ala His Tyr
                165                 170                 175

Lys Ala Cys Leu Phe Ala Gly Val Asn Ile Ser Gly Ile Asn Gly Glu
            180                 185                 190

Val Met Pro Gly Gln Trp Glu Phe Gln Val Gly Pro Thr Val Gly Ile
        195                 200                 205

Ser Ala Gly Asp Gln Val Trp Val Ala Arg Tyr Leu Leu Glu Arg Ile
    210                 215                 220

Thr Glu Ile Ala Gly Val Val Val Thr Phe Asp Pro Lys Pro Ile Pro
225                 230                 235                 240

Gly Asp Trp Asn Gly Ala Gly Ala His Thr Asn Tyr Ser Thr Glu Ser
                245                 250                 255

Met Arg Lys Asp Gly Gly Phe Lys Val Ile Val Asp Ala Val Glu Lys
            260                 265                 270
```

Leu Lys Leu Lys His Lys Glu His Ile Ala Ala Tyr Gly Glu Gly Asn
        275                 280                 285

Glu Arg Arg Leu Thr Gly Lys His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
            340                 345                 350

Leu Trp Lys Pro
        355

<210> 23
<211> 356
<212> PRT
<213> Secale cereale x Triticum turgidum subsp. durum

<400> 23

Met Ala Leu Leu Thr Asp Leu Leu Asn Leu Asp Leu Thr Asp Ser Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Pro Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Lys Gly Asn Asn Ile Leu Val Met Cys Asp Cys Tyr Thr
                85                  90                  95

Pro Ala Gly Val Pro Ile Pro Thr Asn Lys Arg Tyr Asn Ala Ala Lys
            100                 105                 110

Ile Phe Ser Asn Pro Asp Val Ala Lys Glu Glu Pro Trp Tyr Gly Ile
        115                 120                 125

Glu Gln Glu Tyr Thr Leu Leu Gln Lys Asp Ile Asn Trp Pro Leu Gly
    130                 135                 140

Trp Pro Val Gly Gly Phe Pro Gly Pro Gln Gly Pro Tyr Tyr Cys Ser
145                 150                 155                 160

Ile Gly Ala Asp Lys Ser Phe Gly Arg Asp Ile Val Asp Ser His Tyr
                165                 170                 175

Lys Ala Cys Leu Phe Ala Gly Val Asn Ile Ser Gly Ile Asn Gly Glu
            180                 185                 190

Val Met Pro Gly Gln Trp Glu Phe Gln Val Gly Pro Thr Val Gly Ile
        195                 200                 205

Ser Ala Gly Asp Gln Val Trp Val Ala Arg Tyr Leu Leu Glu Arg Ile
    210                 215                 220

Thr Glu Ile Ala Gly Val Val Val Thr Phe Asp Pro Lys Pro Ile Pro
225                 230                 235                 240

Gly Asp Trp Asn Gly Ala Gly Ala His Thr Asn Tyr Ser Thr Glu Ser
                245                 250                 255

Met Arg Lys Asp Gly Gly Phe Lys Val Ile Val Asp Ala Val Glu Lys
            260                 265                 270

Leu Lys Leu Lys His Lys Glu His Ile Ala Ala Tyr Gly Glu Gly Asn
        275                 280                 285

Glu Arg Arg Leu Thr Gly Lys His Glu Thr Ala Asp Ile Asn Thr Phe
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Thr
            340                 345                 350

Leu Trp Lys Pro
        355

<210>  24
<211>  356

<212> PRT
<213> Lolium perenne

<400> 24

Met Ala Leu Leu Thr Asp Leu Leu Asn Leu Asp Leu Ser Gly Ser Thr
1               5                   10                  15

Glu Lys Ile Ile Ala Glu Tyr Ile Trp Ile Gly Gly Ser Gly Met Asp
            20                  25                  30

Leu Arg Ser Lys Ala Arg Thr Leu Pro Gly Pro Val Ser Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Arg Gly Asn Asn Ile Leu Val Met Cys Asp Cys Tyr Thr
                85                  90                  95

Pro Ala Gly Glu Pro Ile Pro Thr Asn Lys Arg Asn Ala Ala Ala Lys
            100                 105                 110

Ile Phe Ser Asn Pro Ala Val Ala Ala Glu Glu Pro Trp Tyr Gly Ile
        115                 120                 125

Glu Gln Glu Tyr Thr Leu Leu Gln Lys Asp Ile Asn Trp Pro Leu Gly
    130                 135                 140

Trp Pro Val Gly Gly Phe Pro Gly Pro Gln Gly Pro Tyr Tyr Cys Ser
145                 150                 155                 160

Ile Gly Ala Glu Lys Ser Phe Gly Arg Asp Ile Val Asp Ser His Tyr
                165                 170                 175

Lys Ala Cys Leu Phe Ala Gly Ile Asn Ile Ser Gly Ile Asn Gly Glu
            180                 185                 190

Val Met Pro Gly Gln Trp Glu Phe Gln Val Gly Pro Ser Val Gly Ile
        195                 200                 205

Ser Ala Gly Asp Gln Val Trp Val Ala Arg Tyr Ile Leu Glu Arg Ile
    210                 215                 220

Thr Glu Ile Ala Gly Val Val Val Thr Phe Asp Pro Lys Pro Ile Pro
225                 230                 235                 240

```
Gly Asp Trp Asn Gly Ala Gly Ala His Thr Asn Tyr Ser Thr Glu Ser
                245                 250                 255

Met Arg Lys Asp Gly Gly Phe Glu Val Ile Val Ala Ala Val Glu Lys
            260                 265                 270

Leu Lys Leu Arg His Lys Glu His Ile Ala Ala Tyr Gly Glu Gly Asn
        275                 280                 285

Glu Arg Arg Leu Thr Gly Lys His Glu Thr Ala Asp Ile His Thr Phe
    290                 295                 300

Ser Trp Gly Val Ala Asn Arg Gly Ala Ser Ile Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ala Met Ile Ala Asp Thr Thr Leu
            340                 345                 350

Leu Trp Lys Pro
        355


<210>  25
<211>  356
<212>  PRT
<213>  Secale cereale

<400>  25

Met Ala Leu Leu Thr Asp Leu Leu Asn Leu Asp Leu Ser Gly Ser Thr
1               5                   10                  15

Asp Lys Ile Ile Ala Glu Tyr Ile Trp Ile Gly Gly Ser Gly Met Asp
            20                  25                  30

Leu Arg Ser Lys Ala Arg Thr Leu Pro Gly Pro Val Thr Asp Pro Ser
        35                  40                  45

Lys Leu Pro Lys Trp Asn Tyr Asp Gly Ser Ser Thr Gly Gln Ala Pro
    50                  55                  60

Gly Glu Asp Ser Glu Val Ile Leu Tyr Pro Gln Ala Ile Phe Lys Asp
65                  70                  75                  80

Pro Phe Arg Lys Gly Asn Asn Ile Leu Val Met Cys Asp Cys Tyr Thr
                85                  90                  95
```

```
Pro Ala Gly Glu Pro Ile Pro Thr Asn Lys Arg His Asn Ala Ala Lys
            100                 105                 110

Ile Phe Ser Asn Pro Asp Val Ala Lys Glu Glu Pro Trp Tyr Gly Ile
        115                 120                 125

Glu Gln Glu Tyr Thr Leu Leu Gln Lys Asp Ile Asn Trp Pro Leu Gly
    130                 135                 140

Trp Pro Val Gly Gly Phe Pro Gly Pro Gln Gly Pro Tyr Tyr Cys Gly
145                 150                 155                 160

Ile Gly Ala Asp Lys Ser Phe Gly Arg Asp Ile Val Asp Ser His Tyr
                165                 170                 175

Lys Ala Cys Met Phe Ala Gly Val Asn Ile Ser Gly Ile Asn Gly Glu
            180                 185                 190

Val Met Pro Gly Gln Trp Glu Phe Gln Val Gly Pro Thr Val Gly Ile
        195                 200                 205

Ser Ala Gly Asp Gln Val Trp Val Ala Arg Tyr Ile Leu Glu Arg Ile
    210                 215                 220

Thr Glu Ile Ala Gly Val Val Val Thr Phe Asp Pro Lys Pro Ile Pro
225                 230                 235                 240

Gly Asp Trp Asn Gly Ala Gly Ala His Thr Asn Tyr Ser Thr Glu Ser
                245                 250                 255

Met Arg Asn Asp Gly Gly Phe Lys Val Ile Val Asp Ala Val Glu Lys
            260                 265                 270

Leu Lys Leu Lys His Lys Glu His Ile Ala Ala Tyr Gly Glu Gly Asn
        275                 280                 285

Glu Arg Arg Leu Thr Gly Lys His Glu Thr Ala Asp Ile Asn Thr Leu
    290                 295                 300

Ser Trp Gly Val Ala Asn Arg Gly Ala Ser Val Arg Val Gly Arg Glu
305                 310                 315                 320

Thr Glu Gln Asn Gly Lys Gly Tyr Phe Glu Asp Arg Arg Pro Ala Ser
                325                 330                 335

Asn Met Asp Pro Tyr Val Val Thr Ser Met Ile Ala Glu Thr Thr Ile
```

340 345 350

Leu Trp Lys Pro
355

<210> 26
<211> 342
<212> PRT
<213> Aegilops tauschii

<400> 26

Met Asp Val Pro Leu Pro Tyr Ser Tyr Arg Gln Ile Gly Gly Ser Gly
1 5 10 15

Met Asp Leu Arg Ser Lys Ala Arg Thr Leu Pro Gly Pro Val Thr Asp
20 25 30

Pro Ser Lys Leu Pro Lys Trp Asn Tyr Asp Gly Ser Ser Thr Gly Gln
35 40 45

Ala Pro Gly Glu Asp Ser Glu Val Ile Leu Tyr Pro Gln Ala Ile Phe
50 55 60

Lys Asp Pro Phe Arg Lys Gly Asn Asn Ile Leu Val Met Cys Asp Cys
65 70 75 80

Tyr Thr Pro Ala Gly Val Pro Ile Pro Thr Asn Lys Arg Tyr Asn Ala
85 90 95

Ala Lys Ile Phe Ser Asn Pro Asp Val Ala Lys Glu Glu Pro Trp Tyr
100 105 110

Gly Ile Glu Gln Glu Tyr Thr Leu Leu Gln Lys Asp Ile Asn Trp Pro
115 120 125

Leu Gly Trp Pro Val Gly Gly Phe Pro Gly Pro Gln Gly Pro Tyr Tyr
130 135 140

Cys Ser Ile Gly Ala Asp Lys Ser Phe Gly Arg Asp Ile Val Asp Ser
145 150 155 160

His Tyr Lys Ala Cys Leu Phe Ala Gly Val Asn Ile Ser Gly Ile Asn
165 170 175

Gly Glu Val Met Pro Gly Gln Trp Glu Phe Gln Val Gly Pro Thr Val
180 185 190

Gly Ile Ser Ala Gly Asp Gln Val Trp Val Ala Arg Tyr Leu Leu Glu

195 200 205

Arg Ile Thr Glu Ile Ala Gly Val Val Val Thr Phe Asp Pro Lys Pro
210 215 220

Ile Pro Gly Asp Trp Asn Gly Ala Gly Ala His Thr Asn Tyr Ser Thr
225 230 235 240

Glu Ser Met Arg Lys Asp Gly Gly Phe Lys Val Ile Val Asp Ala Val
245 250 255

Glu Lys Leu Lys Leu Lys His Lys Glu His Ile Ala Ala Tyr Gly Glu
260 265 270

Gly Asn Glu Arg Arg Leu Thr Gly Lys His Glu Thr Ala Asp Ile Asn
275 280 285

Thr Phe Ser Trp Gly Val Ala Asn Arg Gly Ala Ser Val Arg Val Gly
290 295 300

Arg Glu Thr Glu Gln Asn Gly Lys Gly Tyr Phe Glu Asp Arg Arg Pro
305 310 315 320

Ala Ser Asn Met Asp Pro Tyr Val Val Thr Ser Met Ile Ala Glu Thr
325 330 335

Thr Ile Leu Trp Lys Pro
340

<210> 27
<211> 1571
<212> DNA
<213> Flaveria trinervia

<400> 27
aagctttact cctctcaact ttcaaatcat aacataaaag ttcgtaggtt tgtgttcttc 60

ccaaaaaaaa agtgattttt tttcatcggt taattcatga ttaacatttc gacattcatt 120

ccactatttc acatcatgtt ttgatgggag attgaaatag cgataaggcg aatgtgaaag 180

tgtgaaacag gatgagccac accatcacca catcacaatt tacccaaata atatcccaaa 240

gattcatacg cattttgatc cactgaaacc ccatccaatt ctatccaatg cccaccacat 300

gttcgacgat ttgcctcagt gaatcaagac caacacatgc cactgctttc tgctttttag 360

tccctgataa caaacgattg gctttcattg ctcactgtag aaagtggaga cacccaacaa 420

ctatcatctc cacgtggttc cgtgccgcct ttttgccttt catactgctg gtgcgtcatt 480

tgtcgtcatc aaagcactca cccactatca ttgatctcga aatcttgaat ctttaggttt 540

```
ttatgctttg atacttgaac tctacacaca gtctcgtatc tgactttttg ttatctgtgt      600
tttgctttac taaagatctc acctttaatc aagttttgaa cttttgatgg atttgtcatg      660
ataatgaaga acacatagtt attattgatt atattttgac gaatcttttt tcatgatcgt      720
taaacataat ttgagttctt tttaccttgt ctttctttga ggtttaactg tacatgaaga      780
ctgtattttg agtttattgc ataaatggtc tatatagttt gggttaaaac aactggtttt      840
aatatcaagt ttgatactag acaaaccaac tttttgatta acttttaaaa aaattaataa      900
gtctatttgg aaaaaaattg aaaatttgat tttaaagggt taaaagttct ttttgaaaag      960
ttaataagag taacttttga aatgtaactt ttaaaaaaat actgttgata aaaaaagaaa     1020
tcctaatcat gggcttagta ttgtaagtag cttggatatt gaagctaatt tttcacttta     1080
tatttataga aaagttaatg gaagtaagag gtttggatac tttttttctt aatttagacg     1140
aatgttacac atgaaaaata agcgttgttt tgtaagattt ttttaattcg caagcactaa     1200
actcctaatc aacttttggg gttaaggagt aggcagtaaa ccaaaagcgt ttttgcacga     1260
tacgatgttc aaacatttga tctataacga taagtccaag tgcgttacaa aatgaaactt     1320
tggtatccaa tatgaaactg ggtgtgtagt tcagtaccaa aagcataact ttcagcctcc     1380
ttagtgactt atgactaggc aagagaacat gtgagcccaa tgtactaact ttttacccct     1440
tttattacca ccaccccagc cccccaccat gaaccgatca gaaaaagaag caagaaaaac     1500
agagcattct tgctccttct tcttcatcaa ttcaataaca ttcttcatac cattagaccc     1560
catcttacac t                                                          1571
```